(19) Europäisches Patentamt
European Patent Office
Office européen des brevets

(11) EP 3 662 906 A1

(12) EUROPEAN PATENT APPLICATION
published in accordance with Art. 153(4) EPC

(43) Date of publication:
**10.06.2020 Bulletin 2020/24**

(21) Application number: **17919792.6**

(22) Date of filing: **16.11.2017**

(51) Int Cl.:
***A61K 31/353*** *(2006.01)* ***A61P 3/04*** *(2006.01)*
***A61P 3/06*** *(2006.01)* ***A61P 7/02*** *(2006.01)*
***A61P 9/10*** *(2006.01)* ***A61P 21/00*** *(2006.01)*
***A61P 29/00*** *(2006.01)* ***A61P 35/00*** *(2006.01)*
***A61P 35/02*** *(2006.01)* ***A61P 37/04*** *(2006.01)*
***A61P 37/08*** *(2006.01)* ***A61P 43/00*** *(2006.01)*
***A23L 33/105*** *(2016.01)* ***C12N 15/09*** *(2006.01)*

(86) International application number:
**PCT/JP2017/041248**

(87) International publication number:
**WO 2019/026302 (07.02.2019 Gazette 2019/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **04.08.2017 JP 2017151708**

(71) Applicant: **Kyushu University, National University Corporation**
**Fukuoka-shi, Fukuoka 819-0395 (JP)**

(72) Inventor: **TACHIBANA, Hirofumi**
**Fukuoka-shi, Fukuoka 812-0395 (JP)**

(74) Representative: **EP&C**
**P.O. Box 3241**
**2280 GE Rijswijk (NL)**

(54) **AGONIST OF 67 KDA LAMININ RECEPTOR AND UTILIZATION THEREOF**

(57) A 67 kDa laminin receptor agonist containing an oolong tea polymerized polyphenol or a derivative thereof as an active ingredient.



Printed by Jouve, 75001 PARIS (FR)

## Description

TECHNICAL FIELD

The present invention relates to a 67 kDa laminin receptor (hereinafter sometimes referred to as "67LR") agonist and an use thereof. More specifically, the present invention relates to a 67LR agonist, a 67LR agonist composition, a food composition for preventing or ameliorating a 67LR-related disease, and a method for preventing or ameliorating a 67LR-related disease. Priority is claimed on Japanese Patent Application No. 2017-151708 filed on August 4, 2017, the content of which is incorporated herein by reference.

BACKGROUND ART

Epigallocatechin-O-gallate (hereinafter sometimes referred to as "EGCG") which is one of the main catechins included in green tea has been reported to have an anti-cancer action (see, for example, Non-Patent Document 1), and a phase II clinical test has been conducted in patients with chronic lymphocytic leukemia which is one of hematological cancers (see, for example, Non-Patent Document 2).

The present inventors have hitherto revealed that EGCG exerts an anti-cancer action by binding to 67LR on the cell membrane (see, for example, Non-Patent Document 3). Expression of 67LR is abnormally elevated in cancer cells. In a case where EGCG binds to 67LR, Akt is activated, an endothelial nitric oxide synthase (eNOS) is activated, production of nitric oxide (NO) and cyclic guanosine monophosphate (cGMP) is induced, a protein kinase Cδ (PKCδ) is activated, an acid sphingomyelinase (ASM) is activated, and as a result, cell death is induced. That is, EGCG activates an eNOS/PKCδ/ASM pathway via 67LR and exhibits a cell killing activity.

As the action of EGCG via 67LR, not only an anti-cancer action but also an anti-allergic action, an anti-obesity action, an anti-arteriosclerosis action, an anti-inflammatory action, a muscular atrophy inhibitory action, a cholesterol lowering action, an anti-thrombotic action, an immune enhancing action, a nerve cell protecting action, and the like are known (see, for example, Patent Documents 1 and 2).

Citation List

Patent Literature

[Patent Document 1] PCT International Publication No. WO 2011/162320
[Patent Document 2] PCT International Publication No. WO 2015/199169

Non-Patent Literature

[Non-Patent Document 1] Khan N, et al., Targeting multiple signaling pathways by green tea polyphenol (-)-epigallocatechin-3-gallate., Cancer res., 66 (5), 2500-2505, 2006.
[Non-Patent Document 2] Shanafelt TD, et al., Phase I trial of daily oral Polyphenon E in patients with asymptomatic Rai stage 0 to II chronic lymphocytic leukemia., J. Clin.Oncol., 27 (23), 3808-3814, 2009.
[Non-Patent Document 3] Tachibana H, et al., A receptor for green tea polyphenol EGCG., Nat.Struct. Mol. Biol., 11 (4), 380-381, 2004.

DISCLOSURE OF INVENTION

Technical Problem

Under such circumstances, there is a strong demand for enhancement of the action of EGCG. It is an object of the present invention to provide a 67LR agonist which is more potent than EGCG.

Solution to Problem

The present invention includes the following aspects.

[1] A 67LR agonist containing an oolong tea polymerized polyphenol or a derivative thereof as an active ingredient.
[2] The 67LR agonist according to [1], in which the oolong tea polymerized polyphenol or a derivative thereof is a compound represented by Formula (1) or (2),

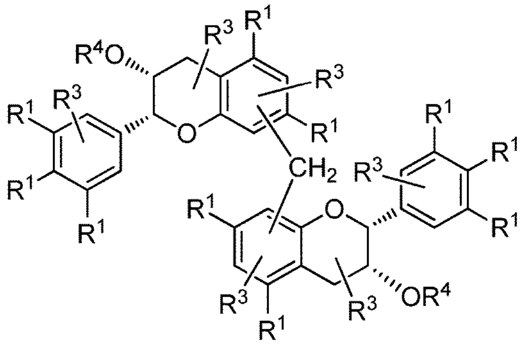

(1)

[in Formula (1), each of $R^1$ independently represents a hydrogen atom, a hydroxyl group, an alkyl group having 1 to 3 carbon atoms or $-OR^2$ (in which $R^2$ represents a group formed by removing one hydrogen atom from glucose, mannose, xylose, galactose, or maltose), each of $R^3$ is independently absent or represents a halogen atom or an alkyl group having 1 to 3 carbon atoms, and each of $R^4$ independently represents a hydrogen atom or a group represented by Formula (1A)]

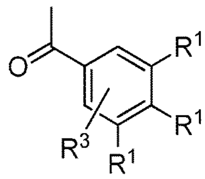

(1A)

[in Formula (1A), $R^1$ and $R^3$ have the same definitions as in Formula (1)]

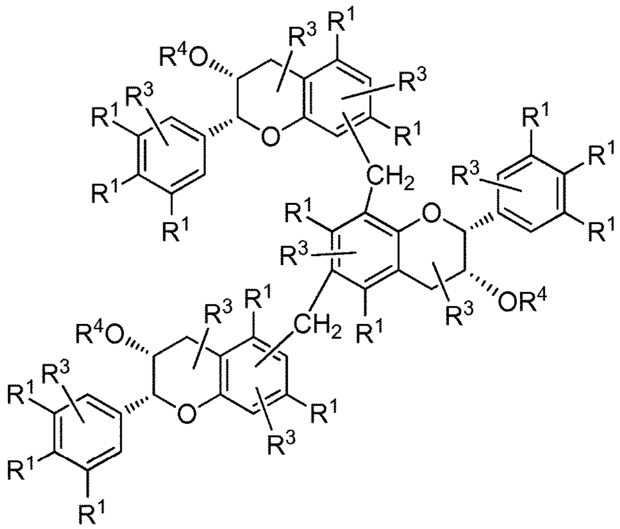

(2)

[in Formula (2), $R^1$, $R^3$, and $R^4$ have the same definitions as in Formula (1)].
[3] The 67LR agonist according to [1] or [2], in which the oolong tea polymerized polyphenol or a derivative thereof is a compound represented by any of Formulae (3) to (6),

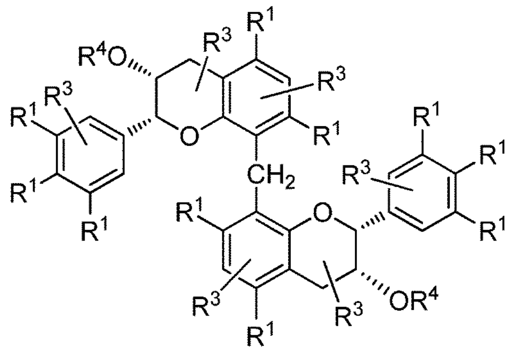

(3)

[in Formula (3), $R^1$, $R^3$, and $R^4$ have the same definitions as in Formula (1)]

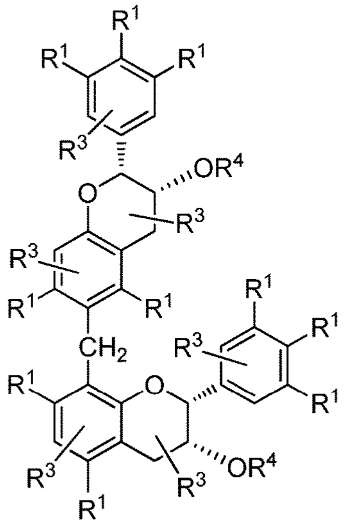

(4)

[in Formula (4), $R^1$, $R^3$, and $R^4$ have the same definitions as in Formula (1)]

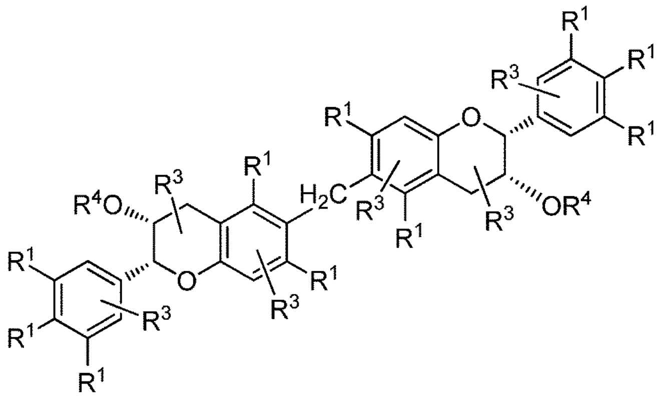

(5)

[in Formula (5), $R^1$, $R^3$, and $R^4$ have the same definitions as in Formula (1)]

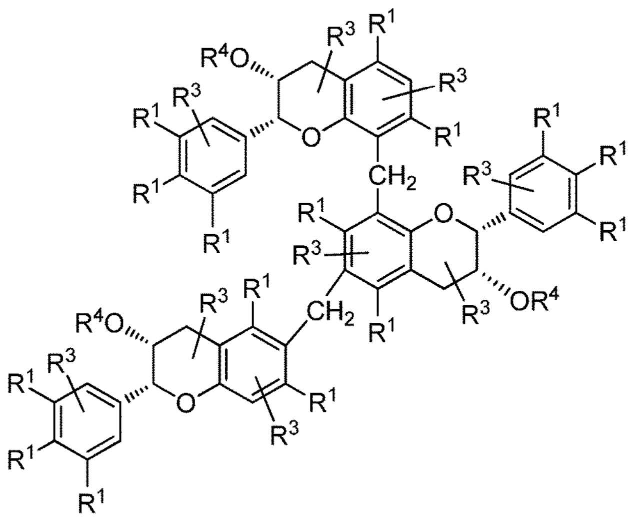

(6)

[in Formula (6), $R^1$, $R^3$, and $R^4$ have the same definitions as in Formula (1)].

[4] The 67LR agonist according to any one of [1] to [3], which is used for preventing or treating a 67LR-related disease.

[5] The 67LR agonist according to [4], in which the 67LR-related disease is cancer, allergy, obesity, arteriosclerosis, inflammation, muscular atrophy, hypercholesterolemia, thrombosis, or immunodeficiency.

[6] A 67LR agonist composition containing the 67LR agonist according to any one of [1] to [3], and a phosphodiesterase inhibitor, or a flavanone or a glycoside thereof.

[7] The 67LR agonist composition according to [6], which is used for preventing or treating a 67LR-related disease.

[8] The 67LR agonist composition according to [7], in which the 67LR-related disease is cancer, allergy, obesity, arteriosclerosis, inflammation, muscular atrophy, hypercholesterolemia, thrombosis or immunodeficiency.

[9] A food composition for preventing or ameliorating a 67LR-related disease, containing the 67LR agonist according to any one of [1] to [3].

[10] The food composition for preventing or ameliorating a 67LR-related disease according to [9], further containing a phosphodiesterase inhibitor, or a flavanone or a glycoside thereof.

[11] A method for preventing or ameliorating a 67LR-related disease, including a step of ingesting the food composition according to [9] or [10] into a human or animal (excluding medical practice on humans).

[12] The method for preventing or ameliorating according to [11], in which the 67LR-related disease is cancer, allergy, obesity, arteriosclerosis, inflammation, muscular atrophy, hypercholesterolemia, thrombosis, or immunodeficiency.

Advantageous Effects of Invention

According to the present invention, a 67LR agonist which is more potent than EGCG can be provided.

BRIEF DESCRIPTION OF DESCRIPTION

In FIG. 1, (a) to (n) are graphs showing survival rates of the respective cancer cell lines in Experimental Example 1.

FIG. 2 is a graph in which an $IC_{50}$ of oolong homobisflavan B (OHBFB) and an $IC_{50}$ of EGCG are plotted against the respective cancer cell lines in Experimental Example 1.

In FIG. 3, (a) and (b) are fluorescence micrographs in Experimental Example 2.

FIG. 4 is a graph showing the measurement results of a cell viability in Experimental Example 3.

FIG. 5 is a graph showing the measurement results of an intracellular cGMP in Experimental Example 4.

FIG. 6 is a graph showing the measurement results of a cell viability in Experimental Example 5.

In FIG. 7, (a) is a photograph showing the results obtained by detecting phosphorylated $eNOS^{Ser1177}$ and eNOS by a Western blotting method in Experimental Example 6. (b) is a graph showing the results obtained by quantifying the results of (a) and calculating the amount of phosphorylated $eNOS^{Ser1177}$ with respect to the amount of eNOS.

FIG. 8 is a graph showing the measurement results of a cell viability in Experimental Example 7.

FIG. 9 is a graph showing the measurement results of an Akt activity in Experimental Example 8.

FIG. 10 is a graph showing the measurement results of a cell viability in Experimental Example 9.

FIG. 11 is a graph showing the measurement results of a cell viability in Experimental Example 10.
FIG. 12 is a graph showing the results of isobologram analysis in Experimental Example 11.
FIG. 13 is a graph showing the results of isobologram analysis in Experimental Example 12.
FIG. 14 is a graph showing the measurement results of a cell viability in Experimental Example 13.
In FIG. 15, (a) and (b) are fluorescence micrographs showing the results obtained by immunostaining CV-caspase-3 in Experimental Example 14. (c) is a graph showing the results obtained by counting the number of CV-caspase-3 positive cells in (a) and (b).
In FIG. 16, (a) and (b) are fluorescence micrographs showing the results obtained by immunostaining phosphorylated $eNOS^{Ser1177}$ in Experimental Example 14.
In FIG. 17, (a) and (b) are fluorescence micrographs showing the results obtained by immunostaining phosphorylated $PKC\delta^{Ser662}$ in Experimental Example 14.
FIG. 18 is a graph showing the measurement results of an ASM activity in tumor tissues of an OHBFB non-administered group (control) and an OHBFB administered group in Experimental Example 14.
FIG. 19 shows a schematic view of an eNOS/PKCδ/ASM pathway activated by OHBFB.
FIG. 20 is a graph showing the measurement results of an intracellular cGMP in Experimental Example 16.
In FIG. 21, (a) to (c) are graphs showing the results of a real-time PCR in Experimental Example 17.
FIG. 22 is a graph showing the results of a real-time PCR in Experimental Example 18.
FIG. 23 is a graph showing the results of a real-time PCR in Experimental Example 19.
FIG. 24 is a graph showing the calculation results of an amount of a TNF-α in Experimental Example 20.
FIG. 25 is a graph showing the calculation results of a release rate of a β-hexosaminidase in Experimental Example 21.

BEST MODE FOR CARRYING OUT THE INVENTION

[67LR Agonist]

In one embodiment, the present invention provides a 67LR agonist containing an oolong tea polymerized polyphenol or a derivative thereof as an active ingredient.
As described later in Examples, the 67LR agonist of the present embodiment activates a signaling pathway downstream of 67LR more potently than EGCG. Here, the expression, being more potent than EGCG, means, for example, that a 50% inhibitory concentration ($IC_{50}$) is lower than that of EGCG. The $IC_{50}$ can be measured, for example, by adding a 67LR agonist serially diluted to a cancer cell medium and measuring a cell viability after 96 hours of culture.
The oolong tea polymerized polyphenol is a generic term for compounds in which catechins are intricately bound and formed by an enzyme reaction or a thermal polymerization reaction in a unique production method of oolong tea, referred to as semi-fermentation, and examples of the oolong tea polymerized polyphenol include dimers of catechins and trimers of catechins. Examples of the dimers of catechins include oolong homobisflavans such as an oolong homobisflavan A, a monodesgalloyl oolong homobisflavan A, an oolong homobisflavan B, and an oolong homobisflavan C, which will be described below.
The molecular weight of the 67LR agonist of the present embodiment may be, for example, 800 or more, for example, 900 or more, or for example, 1,000 or more. The upper limit of the molecular weight of the 67LR agonist is not particularly limited, but may be approximately 3,000.
Generally, it is considered that a compound having a molecular weight of 500 or more has poor absorption into a living body in a case where it is orally administered. For this reason, it is considered that the oolong tea polymerized polyphenol is hardly absorbed into a living body even in a case where it is orally administered.
In contrast, the present inventors have found that the 67LR agonist of the present embodiment reaches a cancer tissue in a living body by oral administration in spite of its relatively large molecular weight and can activate a signaling pathway downstream of 67LR, as described later in Examples.
The 67LR agonist of the present embodiment may be a solvate of the oolong tea polymerized polyphenol or a derivative thereof, may be a pharmaceutically acceptable salt of the oolong tea polymerized polyphenol or a derivative thereof, and may be a solvate of the pharmaceutically acceptable salt.
Examples of the pharmaceutically acceptable salt include inorganic acid salts, alkali metal salts, alkaline earth metal salts, metal salts, ammonium salts, organic amine addition salts, and amino acid addition salts. More specific examples of the pharmaceutically acceptable salt include inorganic acid salts such as hydrochloride, sulfate, hydrobromide, nitrate, and phosphate; organic acid salts such as acetate, mesylate, succinate, maleate, fumarate, citrate, and tartrate; alkali metal salts such as a sodium salt and a potassium salt; alkaline earth metal salts such as a magnesium salt and a calcium salt; metal salts such as an aluminum salt and a zinc salt; ammonium salts such as an ammonium salt and a tetramethylammonium salt; organic amine addition salts such as morpholine and piperidine; and amino acid addition salts such as glycine, phenylalanine, lysine, aspartic acid, and glutamic acid.
Furthermore, the solvate of the oolong tea polymerized polyphenol or a derivative thereof, or the solvate of a

salt of the oolong tea polymerized polyphenol or a derivative thereof is not particularly limited as long as it is a pharmaceutically acceptable solvate, and examples thereof include a hydrate and an organic solvate.

More specific examples of the 67LR agonist of the present embodiment include a compound represented by Formula (1) or (2).

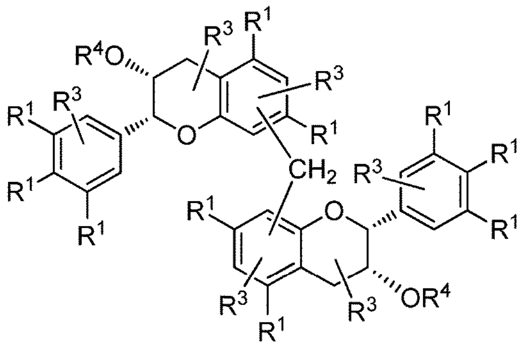

(1)

[in Formula (1), each of $R^1$ independently represents a hydrogen atom, a hydroxyl group, an alkyl group having 1 to 3 carbon atoms or $-OR^2$ (in which $R^2$ represents a group formed by removing one hydrogen atom from glucose, mannose, xylose, galactose, or maltose), each of $R^3$ is independently absent or represents a halogen atom or an alkyl group having 1 to 3 carbon atoms, and each of $R^4$ independently represents a hydrogen atom or a group represented by Formula (1A)].

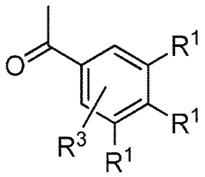

(1A)

[in Formula (1A), $R^1$ and $R^3$ have the same definitions as in Formula (1)].

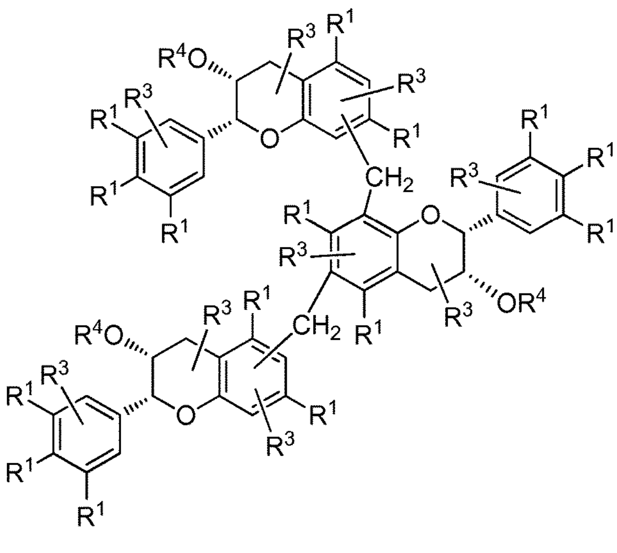

(2)

[in Formula (2), $R^1$, $R^3$, and $R^4$ have the same definitions as in Formula (1)].

The compound represented by Formula (1) includes a compound referred to as oolong homobisflavans, or a derivative thereof. In other words, it can be said that the compound represented by Formula (1) is a compound having dimers of catechins as a skeleton. Furthermore, it can be said that the compound represented by Formula (2) is a compound having trimers of catechins as a skeleton.

The 67LR agonist of the present embodiment may be a derivative of the oolong tea polymerized polyphenol

as long as it has an agonist activity of 67LR. Examples of the derivative of the oolong tea polymerized polyphenol include a compound obtained by modifying a part of a functional group, and the like while the oolong tea polymerized polyphenol being as a basic skeleton.

By derivatizing the oolong tea polymerized polyphenol, it is possible to control, for example, physical properties such as water solubility and oil solubility, and biological activities such as a 67LR agonist activity, a blood retention, and biotoxicity to desired ranges.

Examples of the derivative of the oolong tea polymerized polyphenol include the compound of Formula (1), Formula (1A), or Formula (2), in which each of $R^1$ independently represents a hydrogen atom, a hydroxyl group, an alkyl group having 1 to 3 carbon atoms or $-OR^2$ (in which $R^2$ represents a group formed by removing one hydrogen atom from glucose, mannose, xylose, galactose, or maltose), and each of $R^3$ is independently absent or represent a halogen atom or an alkyl group having 1 to 3 carbon atoms.

Here, examples of the alkyl group having 1 to 3 carbon atoms, represented by $R^1$ or $R^3$, include a methyl group, an ethyl group, an n-propyl group, and an isopropyl group. The alkyl group having 1 to 3 carbon atoms may be substituted. Examples of the substituent of the alkyl group having 1 to 3 carbon atoms include a halogen atom. Examples of the halogen atom include fluorine, fluorine, chlorine, bromine, and iodine. Examples of the halogen atom represented by $R^3$ include fluorine, fluorine, chlorine, bromine, and iodine.

More specific examples of the 67LR agonist of the present embodiment include compounds represented by any of Formulae (3) to (6).

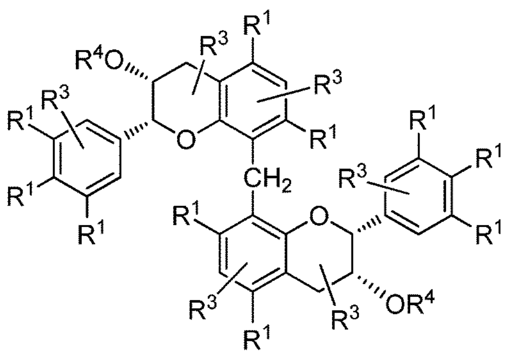

(3)

[in Formula (3), $R^1$, $R^3$, and $R^4$ have the same definitions as in Formula (1)].

A compound represented by Formula (3), in which $R^1$ is a hydroxyl group, $R^3$ is not present, and $R^4$ is represented by Formula (1A), in which $R^1$ is a hydroxyl group and $R^3$ is not present, is a compound known as an oolong homobisflavan A. That is, the 67LR agonist of the present embodiment may be the oolong homobisflavan A or a derivative thereof.

Moreover, in the oolong homobisflavan A, the compound in which one of the groups represented by $R^4$ is a hydrogen atom is a compound known as a monodesgalloyl oolong homobisflavan A. That is, the 67LR agonist of the present embodiment may be the monodesgalloyl oolong homobisflavan A or a derivative thereof.

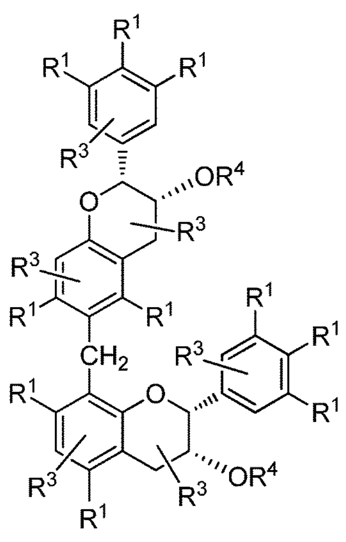

(4)

[in Formula (4), $R^1$, $R^3$, and $R^4$ have the same definitions as in Formula (1)].

A compound represented by Formula (4), in which $R^1$ is a hydroxyl group, $R^3$ is not present, and $R^4$ is represented by Formula (1A), in which $R^1$ is a hydroxyl group and $R^3$ is not present, is a compound known as an oolong homobisflavan B. That is, the 67LR agonist of the present embodiment may be the oolong homobisflavan B or a derivative thereof.

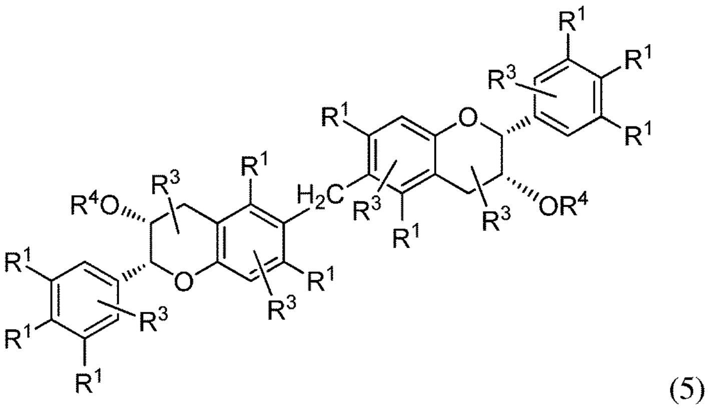

(5)

[in Formula (5), $R^1$, $R^3$, and $R^4$ have the same definitions as in Formula (1)].

A compound represented by Formula (5), in which $R^1$ is a hydroxyl group, $R^3$ is not present, and $R^4$ is represented by Formula (1A), in which $R^1$ is a hydroxyl group and $R^3$ is not present, is a compound known as an oolong homobisflavan C. That is, the 67LR agonist of the present embodiment may be the oolong homobisflavan C or a derivative thereof.

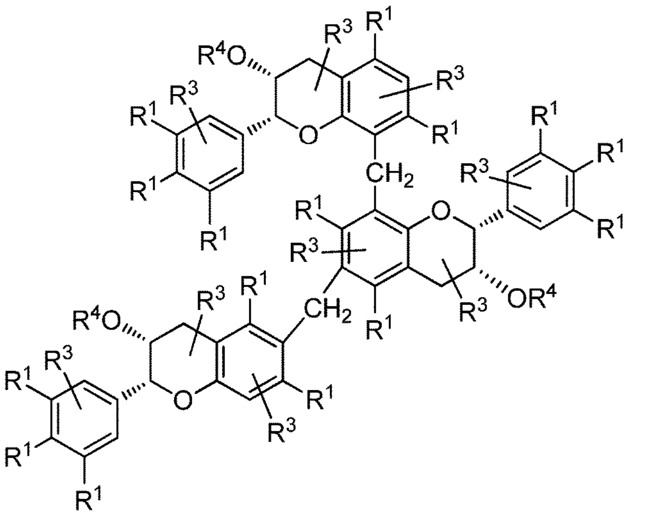

(6)

[in Formula (6), $R^1$, $R^3$, and $R^4$ have the same definitions as in Formula (1)].

The compound represented by Formula (6) can be produced, for example, by the following production method. That is, it can be produced by reacting a flavan-3-ol such as epigallocatechin-3-O-gallate with formaldehyde in a solvent such as methanol and ethanol in the presence of an acid such as hydrochloric acid and sulfuric acid. The concentration of formaldehyde in the reaction solution is preferably 3 to 37 w/v%. The resulting product may be derivatized by esterification, hydrolysis, or the like as necessary.

The 67LR agonist of the present embodiment can be suitably used for preventing or treating a 67LR-related disease. In the present specification, examples of the 67LR-related disease include cancer, allergy, obesity, arteriosclerosis, inflammation, muscular atrophy, hypercholesterolemia, thrombosis, and immunodeficiency.

As described later in Examples, it is possible to exert, for example, a cancer cell killing activity, that is, an anticancer activity by administering the 67LR agonist of the present embodiment to a living body. The cancer is not particularly limited as long as it is a cancer having enhanced expression of 67LR, and examples thereof include multiple myeloma, chronic lymphocytic leukemia, acute myeloid leukemia, chronic myelogenous leukemia, prostate cancer, melanoma, breast cancer, Lung cancer, cervical cancer, and colon cancer.

In addition, as described later in Examples, working mechanism of the 67LR agonist of the present embodiment

is the same as that of EGCG. Therefore, the 67LR agonist of the present embodiment can be used in applications similar to those found in EGCG.

[67LR Agonist Composition]

In one embodiment, the present invention provides a 67LR agonist composition containing the 67LR agonist, and a phosphodiesterase (PDE) inhibitor, or a flavanone or a glycoside thereof, as described above. The 67LR agonist may be used alone or a combination of two or more kinds may be used.
The 67LR agonist composition of the present embodiment may contain the 67LR agonist and the PDE inhibitor as described above.
As described later in Examples, it is possible to synergistically enhance the activity of the 67LR agonist by using the 67LR agonist and the PDE inhibitor as described above in combination.
The PDE inhibitor is a substance which inhibits an enzyme hydrolyzing a cyclic phosphodiesters such as cAMP and cGMP, and a non-selective PDE inhibitor and a selective PDE inhibitor are known. The PDE inhibitor contained in the 67LR agonist composition of the present embodiment may be the non-selective PDE inhibitor or the selective PDE inhibitor.
Examples of the non-selective PDE inhibitor include caffeine, theophylline, theobromine, 3-isobutyl-1-methylxanthine (IBMX), and pentoxifylline.
Examples of the selective PDE inhibitors include a PDE1 inhibitor such as 8-methoxy-3-isobutyl-1-methylxanthine; a PDE2 inhibitor such as erythro-9-(2-hydroxy-3-nonyl)adenine hydrochloride; a PDE3 inhibitor such as cilostamide, milrinone, and trexin; a PDE4 inhibitor such as etazolate hydrochloride and denbufilin; and a PDE5 inhibitor such as vardenafil, sildenafil, zaprinast, tadalafil, dipyridamole, 4-{[3',4'-(methylenedioxy)benzyl]amino}-6-methoxyquinazoline, 1-(3-chloroanilino)-4-phenylphthalazine (MY-5445), and hydrogen sulfide. Among those, the PDE3 inhibitor or the PDE5 inhibitor is preferable.
However, in a case where polysulfides are administered to a living body, they are metabolized to generate hydrogen sulfide. For this reason, it can be said that the polysulfides are the PDE5 inhibitors. Examples of the polysulfides include diallyl disulfide and diallyl trisulfide.
In the 67LR agonist composition of the present embodiment, the PDE inhibitor may be used alone or a combination of two or more kinds may be used. Further, the 67LR agonist may be used alone or a combination of two or more kinds may be used.
In the 67LR agonist composition of the present embodiment, the blend ratio of the 67LR agonist with respect to 100 parts by mass of the PDE inhibitor may be, for example, 1 to 1,000 parts by mass, for example, 1 to 500 parts by mass, for example, 10 to 100 parts by mass, or for example, 10 to 80 parts by mass.
The 67LR agonist composition of the present embodiment may contain the 67LR agonist, and flavanone or a glycoside thereof as described above.
As described later in Examples, it is possible to synergistically enhance the activity of the 67LR agonist by using the 67LR agonist in combination with the flavanone or a glycoside thereof as described above.
Examples of the flavanone or a glycoside thereof (flavanone or a glycoside of flavanone) include eriodictyol, naringenin, hesperetin, eriodictyol glycoside, naringenin glycoside, and hesperetin glycoside.
In the 67LR agonist composition of the present embodiment, the flavanone or a glycoside thereof may be used alone or a combination of two or more kinds may be used. Further, the 67LR agonist may be used alone or a combination of two or more kinds may be used.
In the 67LR agonist composition of the present embodiment, the blend ratio of the 67LR agonist with respect to 100 parts by mass of the flavanone or a glycoside thereof may be, for example, 1 to 100 parts by mass, for example, 10 to 100 parts by mass, or for example, 20 to 100 parts by mass.
The 67LR agonist composition of the present embodiment may contain the 67LR agonist, the PDE inhibitor, and the flavanone or a glycoside thereof as described above.
The 67LR agonist composition of the present embodiment may be used for preventing or treating a 67LR-related disease. Examples of the 67LR-related disease include the same as those described above.

[Pharmaceutical Composition]

The above-mentioned 67LR agonist or 67LR agonist composition may be formulated as a pharmaceutical composition including a pharmaceutically acceptable carrier.
The pharmaceutical composition can be, for example, orally administered in the form of a tablet, a capsule, an elixir, a microcapsule, or the like, or parenterally administered in the form of an injection, a suppository, an external preparation for skin, or the like. More specific examples of the external preparation for skin include a dosage form such as an ointment and a patch.

As the pharmaceutically acceptable carrier, those usually used for the preparation of a pharmaceutical composition can be used without any particular limitation. More specific examples thereof include a binder such as gelatin, corn starch, tragacanth gum, and gum arabic; an excipient such as starch and crystalline cellulose; a leavening agent such as alginic acid; a solvent for an injection, such as water, ethanol, and glycerin; and an adhesive such as a rubber-based adhesive and a silicone-based adhesive.

The pharmaceutical composition may include an additive. Examples of the additive include a lubricant such as calcium stearate and magnesium stearate; a sweetener such as sucrose, lactose, saccharin, and maltitol; a flavorant such as peppermint and red oil; a stabilizer such as benzyl alcohol and phenol; a buffer such as phosphate and sodium acetate; a dissolution aid such as benzyl benzoate and benzyl alcohol; an antioxidant; and a preservative.

The pharmaceutical composition can be formulated by being mixed with the 67LR agonist or 67LR agonist composition as described above, and the pharmaceutically acceptable carrier and additive as described above as appropriate, and being blended into a unit dose form required for generally accepted pharmaceutical practice.

The dose of the pharmaceutical composition varies depending on the condition, the body weight, the age, the sex, and the like of a patient and cannot be unconditionally determined, but in a case of oral administration, the active ingredient (67LR agonist) may be administered at a dose of, for example, 0.1 to 100 mg/kg body weight per dosage unit form. In addition, in a case of the injection, the active ingredient may be administered at a dose of, for example, 0.01 to 50 mg per dosage unit form.

Moreover, a daily dose of the pharmaceutical composition varies depending on the condition, body weight, age, sex, or the like of a patient and cannot be unconditionally determined, but the active ingredient may be administered, for example, once or twice to four times a day at a dose of 0.1 to 100 mg/kg body weight per day for an adult.

[Food Composition]

In one embodiment, the present invention provides a food composition for preventing or ameliorating a 67LR-related disease, containing the above-mentioned 67LR agonist. The food composition of the present embodiment may further contain a PDE inhibitor or a flavanone or a glycoside thereof.

As described later in Examples, the 67LR-related disease can be prevented or ameliorated by ingesting the food composition of the present embodiment into a living body. Examples of the 67LR-related disease include those as described above.

In the food composition of the present embodiment, as the 67LR agonist, those which are approved to be added to food can be used as appropriate.

More specific examples of the 67LR agonist include an oolong tea polymerized polyphenol, an oolong homobisflavan A, a monodesgalloyl oolong homobisflavan A, an oolong homobisflavan B, and an oolong homobisflavan C, which are each extracted from oolong tea.

As the 67LR agonist, chemically synthesized ones or those purified from natural products may be added, or may be contained by using food including the 67LR agonist as a raw material of a food composition. Examples of the food containing the 67LR agonist include oolong tea, an oolong tea extract, black tea, and a black tea extract.

In the food composition of the present embodiment, the PDE inhibitor is preferably a component which is approved to be added to food, and examples thereof include caffeine, theophylline, theobromine, and polysulfides. Examples of the polysulfides include diallyl disulfide and diallyl trisulfide. As the PDE inhibitor, chemically synthesized ones or those purified from natural products may be added, or may be contained by using a food including the PDE inhibitor as a raw material of a food composition. Examples of the food containing caffeine include coffee, green tea, oolong tea, black tea, and cocoa. Examples of the food containing theophylline include tea leaves. Examples of the food containing theobromine include cacao. Examples of the food containing polysulfides include vegetables of genus Allium, such as garlic, spring onion, leek, onion, and Allium bakeri.

In the food composition of the present embodiment, the flavanone or a glycoside thereof is preferably a component which is approved to be added to food, and examples thereof include eriodictyol, naringenin, hesperetin, eriodictyol glycoside, naringenin glycoside, and hesperetin glycoside.

As the flavanone or a glycoside thereof, chemically synthesized ones or those purified from natural products may be added, or may be contained by using food including the flavanone or a glycoside thereof as a raw material of a food composition. Examples of the food containing the flavanone or a glycoside thereof include citrus fruits. Examples of the citrus fruits include the genus Citrus such as orange, grapefruit, Citrus junos, bitter orange, Citrus sphaerocarpa, Citrus sudachi, Citrus yuko hort. ex Tanaka, Yukou (a native Japanese citrus), Citrus depressa, lemon, lime, Citrus natsudaidai, Citrus hassaku, Citrus iyo, Citrus grandis, mandarin orange, satsuma mandarin, Cirus reticulata, Citrus tachibana, Citrus kinokuni, Valencia orange, navel orange, blood orange, Jaffa·orange, bergamot orange, and Chinotto orange; trifoliate oranges; and kumquats.

In the food composition of the present embodiment, each of the 67LR agonist, the PDE inhibitor, and the flavanone or a glycoside thereof may be added alone or a combination of two or more kinds may be used.

The food composition of the present embodiment may be used such that the 67LR agonist may be ingested at 0.1 to 100 mg/kg body weight per day. Further, the food composition may be ingested all at once or divided and taken two to four times a day.

The food composition of the present embodiment may be, for example, in the form of a supplement, may be in the form of a beverage, may be in the form of solid, semi-solid, or gel food, or may be in the form of any prepared food. Examples of the shape of the supplement include the shapes of a capsule and the like used in Examples.

The food composition of the present embodiment may be a food with function claims. The "food with function claims" means food that has been notified to the Consumer Affairs Agency as claiming a function on a product package on a scientific basis. Examples of such the claim include, but are not limited to, "reducing blood cholesterol" and "enhancing innate immunity to contribute to protection from infectious factors." In addition, it is conceivable that even a food composition without function claims is manufactured and sold, with a function being described in a flyer, an advertisement, or the like or being displayed by sound or the like.

The food composition of the present embodiment may be a food for special dietary uses. The food for special dietary uses means a food which is claimed for special dietary uses suitable for the development of infants, toddlers, pregnant women, the sick, and the like, maintenance and recovery of health, and the like under the permission of the government. Alternatively, the food composition of the present embodiment may be a food for the sick among the foods for special dietary uses. Alternatively, the food composition of the present embodiment may be food for specific health uses among the foods for special dietary uses. The specific health food means a food which is approved to be helpful in maintaining and promoting health on a scientific basis and is permitted to claim its effects. The claimed effects and safety are reviewed by a nation and permitted for each food by the Minister of Consumer Affairs Agency.

[Method for Preventing or Ameliorating 67LR-Related Disease]

In one embodiment, the present invention provides a method for preventing or ameliorating a 67LR-related disease (excluding medical practice on humans), including a step of ingesting the food composition described above into a human or an animal. Here, examples of the 67LR-related disease include those described above. In the method for preventing or ameliorating a 67LR-related disease of the present embodiment, the medical practice means practice that a physician (including a person instructed by a physician) carries out for a treatment on a human.

In the method for preventing or ameliorating a 67LR-related disease of the present embodiment, examples of the amount of the food composition to be ingested by a human or an animal include an amount of 0.1 to 100 mg 67LR agonist/kg body weight per day. The food composition may be ingested all at once or divided and taken two to four times a day.

[Other Embodiments]

In one embodiment, the present invention provides a method for treating a 67LR-related disease, including administering to a patient in need thereof an effective amount of an oolong tea polymerized polyphenol or a derivative thereof. Here, examples of the oolong tea polymerized polyphenol or a derivative thereof include the 67LR agonist described above. Examples of the 67LR-related diseases include those described above.

In one embodiment, the present invention provides a method for treating a 67LR-related disease, including administering to a patient in need of treatment a composition containing an oolong tea polymerized polyphenol or derivative thereof and a phosphodiesterase inhibitor, or flavanone or glycoside thereof. Here, the 67LR-related disease, the oolong tea polymerized polyphenol or a derivative thereof, the phosphodiesterase inhibitor, and the flavanone or a glycoside thereof are the same as those described above.

In one embodiment, the present invention provides a method for activating a signaling pathway downstream of 67LR more potently than in a case of bringing EGCG into contact with a 67LR-expressing cell, the method including a step of bringing an oolong tea polymerized polyphenol or a derivative thereof into contact with a 67LR-expressing cell. Here, examples of the oolong tea polymerized polyphenol or a derivative thereof include those described above.

In one embodiment, the present invention provides a method for activating a signaling pathway downstream of 67LR more potently than in a case of bringing EGCG into contact with a 67LR-expressing cell, the method including a step of bringing an oolong tea polymerized polyphenol or a derivative thereof, and a phosphodiesterase inhibitor, or a flavanone or a glycoside thereof into contact with a 67LR-expressing cell. Here, examples of the oolong tea polymerized polyphenol or a derivative thereof, the phosphodiesterase inhibitor, the flavanone, or a glycoside thereof include those described above.

In one embodiment, the present invention provides an oolong tea polymerized polyphenol or a derivative thereof for treatment of a 67LR-related disease. Here, examples of the oolong tea polymerized polyphenol or a derivative thereof include the 67LR agonist described above. Examples of the 67LR-related diseases include those described above.

In one embodiment, the present invention provides a pharmaceutical composition for treating a 67LR-related

disease, containing an oolong tea polymerized polyphenol or a derivative thereof, and a phosphodiesterase inhibitor, or a flavanone or a glycoside thereof. Here, the 67LR-related disease, the oolong tea polymerized polyphenol or a derivative thereof, the phosphodiesterase inhibitor, and the flavanone or a glycoside thereof are the same as those described above.

In one embodiment, the present invention provides a use of an oolong tea polymerized polyphenol or a derivative thereof for production of a drug for preventing or treating a 67LR-related disease. Here, examples of the oolong tea polymerized polyphenol or a derivative thereof include the 67LR agonist described above. Examples of the 67LR-related diseases include those described above.

In one embodiment, the present invention provides a use of an oolong tea polymerized polyphenol or a derivative thereof, and a phosphodiesterase inhibitor, or a flavanone or a glycoside thereof for production of a pharmaceutical composition for preventing or treating a 67LR-related disease. Here, the 67LR-related disease, the oolong tea polymerized polyphenol or a derivative thereof, the phosphodiesterase inhibitor, and the flavanone or a glycoside thereof are the same as those described above.

## EXAMPLES

Next, the present invention will be described in more detail with reference to Experimental Examples, but the present invention is not limited to the following Experimental Examples.

### [Experimental Example 1]

(Anti-Cancer Action of Oolong Homobisflavan B)

The anti-cancer action of Oolong Homobisflavan B (hereinafter sometimes referred to as "OHBFB") was studied. Specifically, OHBFB was added to a medium of various cancer cell lines and the number of viable cells was measured. For comparison, the anti-cancer action of epigallocatechin gallate (hereinafter sometimes referred to as "EGCG") was also studied similarly.

Examples of the cancer cell lines include U266 which is a human multiple myeloma cell line, ARH77 which is a human multiple myeloma cell line, RPMI8226 which is a human multiple myeloma cell line, Mec-1 which is a human chronic lymphocytic leukemia cell line, HL60 which is a human acute myeloid leukemia cell line, Kasumi-1 which is a human acute myeloid leukemia cell line, and KU812 which is human chronic myeloid leukemia cell line were used.

As each of the cancer cell lines, a cell line obtained by subculturing the cells under the conditions of 37°C and 5% $CO_2$ saturated with water vapor using an RPMI 1640 medium supplemented with a 10% fetal calf serum (FCS), and culturing and maintaining the cells in a logarithmic growth phase.

EGCG (manufactured by Sigma-Aldrich) was dissolved in ultrapure water to 5 mM, stored at -30°C, and appropriately thawed before use. OHBFB (manufactured by Nagara Bioscience) was dissolved in ultrapure water to 5 mM, stored at -30°C, and appropriately thawed before use.

Each of the cancer cell lines was suspended in an RPMI 1640 medium supplemented with a 1% FCS, superoxide dismutase (SOD) (5 U/mL), and a catalase (200 U/mL) to $5 \times 10^4$ cells/mL, the suspension was seeded at 100 μL/well in a 96-well plate, OHBFB or EGCG at a final concentration of 0, 5, 10, or 25 μM was added thereto, and the cells were cultured for 96 hours. Thereafter, the number of viable cells was measured using a commercially available kit (trade name "ATPlite," PerkinElmer).

FIG. 1 (a) to (n) are graphs showing the survival rates of the respective cancer cell lines. FIG. 1 (a) to (n) show the results obtained by adding OHBFB to a medium, and FIG. 1 (h) to (n) show the results obtained by adding EGCG to the medium. FIG. 1 (a) and (h) show the results for U266 cells, FIG. 1 (b) and (i) show the results for ARH77 cells, FIG. 1 (c) and (j) show the results for RPMI8226 cells, FIG. 1 (d) and (k) show the results of Mec-1 cells, FIG. 1 (e) and (1) show the results of HL60 cells, FIG. 1 (f) and (m) show the results of Kasumi-1 cells, and FIG. 1 (g) and (n) show the results for KU812 cells.

As a result, it became clear that OHBFB exhibits an anti-cancer action on each of cancer cell lines of U266 ($IC_{50}$ = 4.0 μM), ARH77 ($IC_{50}$ = 0.2 μM), RPMI8226 ($IC_{50}$ = 4.4 μM), Mec-1 ($IC_{50}$ = 1.4 μM), HL60 ($IC_{50}$ = 2.5 μM), Kasumi-1 ($IC_{50}$ = 3.0 μM), and KU812 ($IC_{50}$ = 0.2 μM).

Furthermore, it became clear that EGCG also exhibits an anti-cancer action on each of cancer cell lines of U266 ($IC_{50}$ = 11.8 μM), ARH77 ($IC_{50}$ = 4.0 μM), RPMI8226 ($IC_{50}$ = 11.1 μM), Mec-1 ($IC_{50}$ = 8.3 μM), HL60 ($IC_{50}$ = 9.5 μM), Kasumi-1 ($IC_{50}$ = 9.6 μM), and KU812 ($IC_{50}$ = 6.2 μM).

In addition, from the comparison of $IC_{50}$ between OHBFB and EGCG, it became clear that OHBFB has at least about three times more potent anti-cancer action than EGCG.

Moreover, FIG. 2 is a graph in which the $IC_{50}$ of OHBFB and the $IC_{50}$ of EGCG for each of cancer cell lines are plotted. As a result, it became clear that there is a positive correlation between the sensitivity of each of the cancer

cell lines to OHBFB and the sensitivity of each of the cancer cell lines to EGCG.

In addition, in statistical processing of the experimental results, a Tukey's test was used and a P value of less than 0.05 was regarded as being significant. The same applies to the following Experimental Examples.

[Experimental Example 2]

(OHBFB Induces Change in Lipid Raft Localization)

The effect of OHBFB on lipid rafts of cancer cells was evaluated. Specifically, first, a human multiple myeloma cell line U266 was suspended in an RPMI 1640 medium supplemented with 1% FCS at $1 \times 10^7$ cells/mL and added to a 96-well plate at 200 μL/well.

Subsequently, Alexa Fluor (registered trademark) 555-labeled Cholera Toxin B subunit (manufactured by Thermo Fisher Scientific, Inc.) (1 mg/mL) was added thereto at 3 μL per well, and the cells were stained on ice for 15 minutes. After staining, the supernatant was removed by centrifugation, an RPMI 1640 medium containing OHBFB at a final concentration of 5 μM was added thereto, and the cells were incubated for 3 hours. Subsequently, the resultant was observed with a fluorescence microscope using an oil immersion lens (60 magnification).

As a control, a sample which has been subjected to the same operation as above, except that the same volume of a phosphate buffer (PBS) had been added instead of OHBFB was used. PBS was prepared by dissolving 8.0 g of NaCl, 0.2 g of KCl, 1.15 g of $Na_2HPO_4$, and 0.2 g, $KH_2PO_4$ in 1 L of ultrapure water and autoclaving the solution.

FIG. 3 (a) and (b) are fluorescence micrographs. FIG. 3 (a) is a photograph showing the result of the control sample, and FIG. 3 (b) is a photograph showing the result obtained by adding OHBFB. As a result, it became clear that OHBFB exhibited a lipid raft association action in the same manner to EGCG.

[Experimental Example 3]

(Inhibition of Anti-Cancer Action of OHBFB by Acid Sphingomyelinase (ASM) Inhibitor)

The involvement of an acid sphingomyelinase in the action of OHBFB on reducing the number of viable cells on cancer cells was evaluated.

Specifically, a human multiple myeloma cell line U266 was suspended in an RPMI 1640 medium supplemented with 1% FCS, SOD (5 U/mL), and a catalase (200 U/mL) to $5 \times 10^4$ cells/mL, the suspension was seeded at 1 mL per well in a 24-well plate, Desipramine (manufactured by Sigma-Aldrich) which is an acid sphingomyelinase inhibitor was added thereto to a final concentration of 5 μM, and the cells were incubated for 3 hours. Thereafter, OHBFB was added to a final concentration of 5 μM and the cells were further cultured for 96 hours. Subsequently, the cell viability was measured by trypan blue staining.

Desipramine (manufactured by Sigma-Aldrich) was dissolved in ultrapure water to 5 mM, stored at -30°C, and appropriately thawed before use.

FIG. 4 is a graph showing the measurement results of the cell viability. As a result, it became clear that the cancer cell killing activity of OHBFB was inhibited by the addition of Desipramine. From these results, it became clear that OHBFB exhibits an anti-cancer action in an acid sphingomyelinase-dependent fashion in the same manner to EGCG.

[Experimental Example 4]

(cGMP Production Inducing Ability 1 of OHBFB)

The cGMP production inducing action of OHBFB on cancer cells was evaluated. Specifically, U266 which is a human multiple myeloma cell line was seeded in a 96-well plate at a cell density of $5.0 \times 10^5$ cells/well in an RPMI 1640 medium containing OHBFB at a final concentration of 5 μM and 1% FCS, and the cells were incubated for 3 hours. Thereafter, the amount of cGMP in the cell was measured using a commercially available kit (trade name "cGMP EIA kit," manufactured by Cayman Chemical Co., Ltd.).

FIG. 5 is a graph showing the measurement results of intracellular cGMP. As a result, it became clear that OHBFB had a cGMP production inducing action against cancer cells, in the same manner to EGCG.

[Experimental Example 5]

(Inhibition of Anti-Cancer Action of OHBFB by Inhibitor of Soluble Guanylate Cyclase (sGC) Which Is cGMP Synthase)

The involvement of a soluble guanylate cyclase (sGC) in the action of OHBFB to reduce the number of viable

cells with respect to the cancer cells was evaluated. Specifically, U266 which is a human multiple myeloma cell line was suspended in an RPMI 1640 medium supplemented with 1% FCS, SOD (5 U/mL), and a catalase (200 U/mL) to $5 \times 10^4$ cells/mL, and the suspension was seeded at 1 mL per well in a 24-well plate.

Subsequently, NS2028 (manufactured by Sigma-Aldrich) which is an sGC inhibitor was added thereto to a final concentration of 5 μM, and the cells were incubated for 3 hours. Subsequently, the cell medium was replaced with a medium containing OHBFB at a final concentration of 5 μM and the cells were further cultured for 96 hours. Thereafter, the cell viability was measured by trypan blue staining.

NS2028 (manufactured by Sigma-Aldrich) was dissolved in ultrapure water to 5 mM, stored at -30°C, and appropriately thawed before use.

FIG. 6 is a graph showing the measurement results of the cell viability. As a result, it became clear that the cancer cell killing activity of OHBFB disappeared by the addition of the sGC inhibitor. That is, it became clear that OHBFB exhibits an anti-cancer action in an sGC-dependent fashion in the same manner to EGCG.

[Experimental Example 6]

(Activation of Endothelial Monoxide Synthase (eNOS) by OHBFB)

The effect of OHBFB on eNOS in cancer cells was evaluated. Specifically, U266 which is a human multiple myeloma cell line was suspended in an RPMI 1640 medium supplemented with 1% FCS at $1 \times 10^6$ cells/mL and the suspension was seeded at 1 mL per well in a 24-well plate. Subsequently, OHBFB was added to a final concentration of 5 μM and the cells were cultured for 1 hour. As a control, cells to which OHBFB had not been added were used.

Subsequently, the phosphorylation level of $eNOS^{Ser1177}$ which an indicator of eNOS activation was measured by a Western blotting method using an anti-phosphorylated $eNOS^{Ser1177}$ antibody.

FIG. 7 (a) is a photograph showing the results obtained by detecting phosphorylated $eNOS^{Ser1177}$ and eNOS by a Western blotting method. FIG. 7 (b) is a graph showing the result obtained by quantifying the result of FIG. 7 (a) and calculating the amount of phosphorylated $eNOS^{Ser1177}$ with respect to the amount of eNOS. The results are shown in a percentage (%) with a value in the control being defined as 100.

As a result, it became clear that OHBFB induces eNOSser1177 phosphorylation of cancer cells, that is, activates eNOS, in the same manner to EGCG.

[Experimental Example 7]

(Inhibition of Anti-Cancer Action of OHBFB by eNOS Inhibitor)

The involvement of eNOS in the action of OHBFB on reducing the number of viable cells on cancer cells was evaluated. Specifically, U266 which is a human multiple myeloma cell line was suspended in an RPMI 1640 medium supplemented with 1% FCS, SOD (5 U/mL), and a catalase (200 U/mL) to $5 \times 10^4$ cells/mL, and the suspension was seeded at 1 mL per well in a 24-well plate.

Subsequently, L-NAME (manufactured by Wako Pure Chemical Industries, Ltd.) which is an eNOS inhibitor was added thereto to a final concentration of 10 mM, and the cells were incubated for 3 hours. Subsequently, OHBFB was added to a final concentration of 5 μM and the cells were cultured for 96 hours. Thereafter, the cell viability was measured by trypan blue staining.

L-NAME (manufactured by Wako Pure Chemical Industries, Ltd.) was dissolved in ultrapure water to 1 M, stored at -30°C, and appropriately thawed before use.

FIG. 8 is a graph showing the measurement results of the cell viability. As a result, the cancer cell killing activity of OHBFB was inhibited by the addition of the eNOS inhibitor. That is, it became clear that OHBFB exhibits an anti-cancer action in an eNOS-dependent fashion in the same manner to EGCG.

[Experimental Example 8]

(Studies on Akt Activating Ability of OHBFB)

The Akt activating ability of OHBFB in cancer cells was evaluated. Specifically, U266 which is a human multiple myeloma cell line was suspended in an RPMI 1640 medium supplemented with 1% FCS at $1 \times 10^7$ cells/mL and the suspension was seeded at 1 mL per well in a 24-well plate. Subsequently, OHBFB was added to a final concentration of 5 μM and incubated for 1 hour. As a control, cells to which OHBFB had not been added were used. Subsequently, a cell lysis buffer was added to lyse and recover the cells.

Subsequently, the Akt activity of the recovered sample was measured using a commercially available kit ("K-LISA (trade name) Akt Activity Kit", model "CBA019", manufactured by Merck Millipore).

FIG. 9 is a graph showing the measurement results of the Akt activity. The results are shown in a percentage (%) with a value in the control being defined as 100. As a result, it became clear that OHBFB exhibits an Akt activating action on cancer cells in the same manner to EGCG.

[Experimental Example 9]

(Inhibition of Anti-Cancer Action of OHBFB by Anti-67LR Monoclonal antibody)

The involvement of 67LR in the action of OHBFB on reducing the number of viable cells on cancer cells was evaluated. Specifically, U266 which is a human multiple myeloma cell line was suspended in an RPMI 1640 medium supplemented with 1% FCS, SOD (5 U/mL), and a catalase (200 U/mL) to $5 \times 10^4$ cells/mL, and the suspension was seeded at 100 μL per well in a 96-well plate.

Subsequently, an anti-67LR monoclonal antibody (model "MLuc5," manufactured by Abcam) or a control antibody was added to a final concentration of 20 μg/mL, and the cells were incubated for 3 hours. Subsequently, OHBFB was added to a final concentration of 5 μM and the cells were further cultured for 96 hours. Thereafter, the cell viability was measured by trypan blue staining.

FIG. 10 is a graph showing the measurement results of the cell viability. As a result, it became clear that in the cells pretreated with the anti-67LR monoclonal antibody, the viable cell count reducing action was significantly reduced by adding OHBFB. That is, it became clear that OHBFB exhibits an anti-cancer action in a 67LR-dependent fashion in the same manner to EGCG.

[Experimental Example 10]

(Inhibition of Anti-Cancer Action of OHBFB by Suppressing Expression of 67LR)

The involvement of 67LR in the anti-cancer action of OHBFB was evaluated. Specifically, a mouse melanoma cell line B16 was suspended in a DMEM medium supplemented with 5% FCS to $1 \times 10^4$ cells/mL, and the suspension was seeded at 1 mL per well in a 24-well plate. Subsequently, 67LR siRNA (manufactured by Life Technologies) or a control siRNA (manufactured by Life Technologies) was introduced thereinto and the cells were cultured for 72 hours. For introduction of siRNA, a commercially available kit (trade name "RNAiMAX," manufactured by Thermo Fisher Scientific) was used.

Thereafter, each cell was seeded again at a cell density of $1\times10^4$ cells/mL in a 96-well plate at 100 μL per well and incubated for 24 hours. The medium used was a DMEM medium supplemented with 5% FCS. Subsequently, OHBFB was added to a final concentration of 5 μM and the cells were further cultured for 72 hours. Thereafter, the cell viability was measured by trypan blue staining.

FIG. 11 is a graph showing the measurement results of the cell viability. As a result, it became clear that in the cancer cells in which the expression of 67LR was reduced, the activity of suppressing the growth of cancer cells by the addition of OHBFB was significantly reduced. This result further supports that OHBFB exhibits an anti-cancer action in a 67LR-dependent fashion in the same manner to EGCG.

[Experimental Example 11]

(Anti-Cancer Activity Enhancing Action of OHBFB by Phosphodiesterase (PDE) 5 Inhibitor)

The anti-cancer activity enhancing action of OHBFB by the PDE5 inhibitor was evaluated. Specifically, a human multiple myeloma cell line U266 was suspended in an RPMI 1640 medium supplemented with 1% FCS, SOD (5 U/mL), and a catalase (200 U/mL) to $5 \times 10^4$ cells/mL, and the suspension was seeded at 100 μL per well in a 96-well plate.

Subsequently, vardenafil (manufactured by Cayman Chemical Co.) which is a PDE5 inhibitor was added to a final concentration of 5 μM and the mixture was incubated for 3 hours. Subsequently, OHBFB at final concentrations of 0, 0.5, 1.0 and 2.5 μM was added thereto and the cells were further cultured for 96 hours. Thereafter, the cell viability was measured using a commercially available kit (trade name "ATPlite," PerkinElmer).

As a result, the $IC_{50}$ of OHBFB in a case of a combined use of OHBFB and vardenafil was calculated to be 1.07 μM.

In addition, the same studies as described above were performed in the presence of vardenafil alone at final concentrations of 0, 5, 10, 25, 50, and 100 μM. As a result, the $IC_{50}$ of vardenafil was calculated to be 92.68 μM.

Subsequently, it was studied whether the anti-cancer activity of the combined use of OHBFB and vardenafil was antagonistic, additive, or synergistic by isobologram analysis. FIG. 12 is a graph showing the results of the isobologram analysis.

Specifically, as shown in FIG. 12, the $IC_{50}$ of OHBFB (4.0 μM) was plotted on the x-axis, the $IC_{50}$ of vardenafil (92.68 μM) was plotted on the y-axis, and a straight line connecting these values was drawn. Further, the $IC_{50}$ (1.07 μM) of OHBFB in a case of being used in combination with vardenafil (final concentration of 5 μM) was plotted.

As a result, it became clear that the PDE5 inhibitor synergistically enhances the anti-cancer activity of OHBFB from the viewpoint that the plot in a case of the combined use of OHBFB and vardenafil was closer to the origin point than the straight line.

[Experimental Example 12]

(Anti-Cancer Activity Enhancing Action of OHBFB by Eriodictyol)

The anti-cancer activity enhancing action of OHBFB by eriodictyol was evaluated. Specifically, U266 which is a human multiple myeloma cell line was suspended in an RPMI 1640 medium supplemented with 1% FCS, SOD (5 U/mL), and a catalase (200 U/mL) to $5 \times 10^4$ cells/mL, and the suspension was seeded at 100 μL per well in a 96-well plate.

Subsequently, in the absence of eriodictyol (manufactured by EXTRASYNTHESE) or in the presence of a final concentration of 5 μM, OHBFB at a final concentration of 0, 1.0, 2.5 and 5.0 μM was added, and the cells were cultured for 96 hours. Thereafter, the cell viability was measured using a commercially available kit (trade name "ATPlite," PerkinElmer).

As a result, the $IC_{50}$ of OHBFB when used in combination with OHBFB and eriodictyol was calculated to be 1.16 μM.

In addition, the same study as described above was performed in the presence of eriodictyol alone at final concentrations of 0, 5, 10, 25, and 50 μM. As a result, the IC50 of eriodictyol was calculated to be 89.4 μM.

Subsequently, it was studied by isobologram analysis whether the anti-cancer activity of the combined use of OHBFB and eriodictyol was antagonistic, additive, or synergistic. FIG. 13 is a graph showing the results of isobologram analysis.

Specifically, as shown in FIG. 13, the $IC_{50}$ of OHBFB (4.0 μM) was plotted on the x-axis, the $IC_{50}$ of eriodictyol (89.4 μM) was plotted on the y-axis, and a straight line connecting these values was drawn. Further, the $IC_{50}$ (1.16 μM) of OHBFB in a case of being used in combination with eriodictyol (final concentration of 5 μM) was plotted.

As a result, it became clear that eriodictyol synergistically enhances the anti-cancer activity of OHBFB from the viewpoint that the plot in a case of the combined use of OHBFB and eriodictyol was closer to the origin point than the straight line.

[Experimental Example 13]

(Anti-Cancer Activity Enhancing Action of OHBFB by Flavanone)

The anti-cancer activity enhancing action of OHBFB by flavanone was evaluated. Specifically, U266 which is a human multiple myeloma cell line was suspended in an RPMI 1640 medium supplemented with 1% FCS, SOD (5 U/mL), and a catalase (200 U/mL) to $5 \times 10^4$ cells/mL, and the suspension was seeded at 100 μL per well in a 96-well plate. Subsequently, a flavanone at a final concentration of 5 μM and OHBFB at a final concentration of 0 μM or 2.5 μM were each added thereto and the cells were cultured for 96 hours. As a control, a sample to which the same volume of PBS had been added instead of flavanone was used.

As the flavanone, eriodictyol (manufactured by Extrasynthese Corp.), naringenin (manufactured by Tokyo Chemical Industry Co., Ltd.), and hesperetin (manufactured by Wako Pure Chemical Industries, Ltd.) were used.

Subsequently, the cell viability was measured using a commercially available kit (trade name "ATPlite," PerkinElmer).

FIG. 14 is a graph showing the measurement results of the cell viability. As a result, it became clear that eriodictyol, naringenin, and hesperetin all enhance the anti-cancer activity of OHBFB.

[Experimental Example 14]

(67LR-Dependent Cancer Cell Killing Activity Inducing Ability of Orally Administered OHBFB against Tumor Tissue)

OHBFB was orally administered to mice and the 67LR-dependent cancer cell killing activity inducing ability was evaluated.

Specifically, first, a 5-week-old female Balb/c mouse was preliminarily raised for 2 weeks. Subsequently, 200 μL of a suspension obtained by suspending MPC-11 which is a mouse multiple myeloma cell line in an RPMI 1640

medium to 5 × $10^6$ cells/mL was subcutaneously injected and transplanted into the right back of the mouse. Then, 5 days after transplantation, OHBFB was orally administered at a dose of 10 mg/kg. Subsequently, 5 hours later, the mouse was sacrificed and the tumor was excised therefrom to prepare a tissue section. As a control, a tumor tissue section prepared in the same manner as described above, except that OHBFB had not been administered, was used.

Subsequently, the tumor tissue section was immunostained to detect the presence of Cleaaved (CV)-caspase-3 (CV-caspase-3), phosphorylated $eNOS^{Ser1177}$, and phosphorylated $PKC\delta^{Ser662}$.

«Detection of CV-Caspase-3»

FIG. 15 (a) and (b) are fluorescence micrographs showing the results obtained by immunostaining CV-caspase-3. FIG. 15 (a) is a photograph showing the results obtained by staining the tumor tissue section as a control, and FIG. 15 (b) is a photograph showing the results obtained by staining the tumor tissue section of a mouse to which OHBFB was administered. In addition, FIG. 15 (c) is a graph showing the results obtained by counting the number of CV-caspase-3 positive cells in FIG. 15 (a) and FIG. 15 (b).

As a result, it became clear that the number of CV-caspase-3 positive cells which is an indicator of apoptosis was increased in the tumor tissues of the mouse to which OHBFB had been orally administered.

<<Phosphorylated $eNOS^{Ser1177}$>>

FIG. 16 (a) and (b) are fluorescence micrographs showing the results obtained by immunostaining phosphorylated $eNOS^{Ser1177}$. FIG. 16 (a) is a photograph showing the results obtained by staining the tumor tissue section as a control, and FIG. 16 (b) is a photograph showing the results obtained by staining the tumor tissue section of a mouse to which OHBFB had been administered.

As a result, it became clear that phosphorylated $eNOS^{Ser1177}$ was significantly increased in the tumor tissues of the mouse to which OHBFB had been orally administered.

<<Detection of Phosphorylated $PKC\delta^{Ser662}$>>

FIG. 17 (a) and (b) are fluorescence micrographs showing the results obtained by immunostaining phosphorylated $PKC\delta^{Ser662}$. FIG. 17 (a) is a photograph showing the results obtained by staining the tumor tissue section as a control, and FIG. 17 (b) is a photograph showing the results obtained by staining the tumor tissue section of a mouse to which OHBFB was administered.

As a result, it became clear that phosphorylated $PKC\delta^{Ser662}$ was significantly increased in the tumor tissues of the mouse to which OHBFB had been orally administered.

«Measurement of ASM Activity»

In addition, the acid sphingomyelinase (ASM) activity of the tumor tissue was measured. The ASM activity was measured as follows. The mouse to which OHBFB had been orally administered at a dose of 10 mg/kg were sacrificed 6 hours after the administration of OHBFB, and the tumor tissue was excised. As a control, a tumor tissue excised from a mouse in the OHBFB non-administered group was used.

Subsequently, each of the extracted tumor tissues was crushed and centrifuged, the supernatant was adjusted to 2 mg protein/mL with PBS, and then 20 μL of the supernatant was dispensed into a tube. Subsequently, 8 μL of a substrate mixed liquid (2 μL of BODIPY-C12-sphingomyelin (manufactured by Thermo Fisher Scientific), 10 μL of 10% Triton X-100, 20 μL of 1 M sodium acetate (pH 4.5), and 78 μL of water) was added into the tube, and mixed and incubated at 37°C for 8 hours. As a result of this reaction, BODIPY-labeled ceramide is produced from BODIPY-C12-sphingomyelin by the activity of ASM.

Subsequently, 60 μL of a stop solution (chloroform:methanol = 2:1) was added thereto, and 10 μL of each portion of the solution was spotted on a thin-layer chromatography plate which had been activated in advance by a drier, and developed with a developing solvent (water:methanol:chloroform = 8:35:60).

Subsequently, the thin-layer chromatography plate was irradiated with ultraviolet light, and the fluorescence signal of the BODIPY-labeled ceramide was detected using a fluorescent image analyzer (trade name "Fusion System," manufactured by Bilber-Rumat Co., Ltd.), analyzed using an analysis software (trade name "Kyplot," Keyence), and quantified.

FIG. 18 is a graph showing the measurement results of an ASM activity in the tumor tissues of an OHBFB non-administered group (control) and an OHBFB administered group. The results are shown as a percentage (%) with the measured value of the ASM activity in the control as 100.

As a result, it became clear that the ASM activity was significantly increased in the tumor tissue of the mouse

to which OHBFB had been orally administered.

From the results, it became clear that OHBFB exhibits a 67LR-dependent cancer cell killing activity that involves activation of the eNOS/PKCδ/ASM pathway in a living body, in the same manner to EGCG. FIG. 19 shows a schematic view of the eNOS/PKCδ/ASM pathway activated by OHBFB.

[Experimental Example 15]

(OHBFB Administered Orally is Delivered to Tumor Tissue)

Generally, it is considered that a compound having a molecular weight of 500 or more has poor absorption into a living body in a case where it is orally administered. For this reason, it is considered that the oolong tea polymerized polyphenol is hardly absorbed into a living body even in a case where it is orally administered. Thus, it was studied whether the orally administered OHBFB reaches the tumor tissue.

«Oral Administration of OHBFB and Excision of Tumor Tissue»

First, a Balb/c mice (five-week-old female) was preliminarily raised for two weeks. Subsequently, MPC-11 which is a mouse plasmacytoma cell line was subcutaneously injected and transplanted into the right back at $1 \times 10^6$ cells/mouse.

Subsequently, OHBFB (10 mg/kg) was orally administered (i.g.) to the mouse in which tumor formation had been confirmed, and 5 hours after the administration, the mouse was sacrificed by cardiac blood sampling under isoflurane anesthesia to excise the tumor tissue.

Subsequently, 400 mL of PBS containing 2% ascorbic acid and 5 mL of 100 mM propyl gallate as an internal standard was added to the excised tumor tissue, and the tissue was disrupted with a bead shocker. Subsequently, the crushed tissue was centrifuged, and the supernatant was recovered in a tube and extracted with ethyl acetate. After the extracted sample was solidified by drying, it was redissolved in 100 mL of a 50% acetonitrile solution containing 4-hydroxybenzophenone (4HB) (10 mM) as an internal standard, passed through a 0.22 mm PVDF filter, and then subjected to LC-MS analysis.

<<LC-MS Analysis>>

A sample was subjected to LCMS-IT-TOF (Shimadzu Corporation) at an injection volume of 5 μL. As a mobile phase, (A) ultrapure water containing 0.05% formic acid and (B) acetonitrile containing 0.05% formic acid were used. The flow rate was 0.15 mL/min, and the concentration of mobile phase B was measured under the gradient conditions of 0 to 2 min: 5%, 2 to 3.5 min: 5 to 20%, 3.5 to 10 min: 20 to 40%, 10 to 13 min: 40 to 100%, 13 to 15.5 min 100%, 15.5 to 16 min: 100 to 5%, and 16 to 20 min: 5%. As the column, L-column 2 ODS (2.1 × 150 mm, 3 μm, CERI) was used (column oven: 40°C). That is, the ionization was performed by an Electrospray ionization (ESI) method (negative ion mode), the CDL and the heat block temperature were set to 200°C, and data were obtained in a scan mode (m/z 350 to 1,400).

The precise mass of OHBFB ($C_{45}H_{36}O_{22}$) was m/z 928.169, and the LC-MS analysis (negative ion mode) of the standard product showed that an MS peak was recognized with ion m/z 927.158 $[M-H]^-$ (theoretical value: 927.169) from hydrogen had left.

Thus, as the MS spectrum of the tumor sample was confirmed, such an ion peak was not observed in the OHBFB non-administered group, but a peak with m/z 927.157 was recognized in the OHBFB-administered group. Furthermore, in the MS/MS analysis of this peak, m/z 758.132, 608.113, and 470.071 were detected as major product ions.

From the viewpoint that these detection peaks were consistent with product ions (m/z 758.139, 608.106, 470.081) determined by MS/MS analysis of a standard product of OHBFB, it became clear that OHBFB was present in the tumor tissue after oral administration.

[Experimental Example 16]

(cGMP Production Inducing Ability 2 of OHBFB)

The cGMP production inducing action of OHBFB on cells was evaluated. Specifically, first, HUVEC which is a human umbilical vein endothelial cell was suspended in an EGM-2 medium to be adjusted to $5 \times 10^6$ cells/mL. Subsequently, OHBFB was added thereto to a final concentration of 5 μM and the cells were seeded at 50 μL/well in a 96-well plate. Then, after incubation at 37°C for 1 hour, the amount of cGMP in the cells was measured by an RT-FRET method (cGMP assay, Cisbio).

FIG. 20 is a graph showing the measurement results of intracellular cGMP. In FIG. 20, the data are expressed in a mean value ± a standard deviation (n = 3). In addition, "**" indicates that a statistically significant difference exists at $p < 0.01$ by a Student's t-test.

As a result, it became clear that OHBFB promotes the production of intracellular cGMP for human umbilical vein endothelial cells HUVEC. This result further supports that OHBFB has a cGMP-related physiological action in a similar manner to EGCG.

[Experimental Example 17]

(Immune Function Enhancing Action of OHBFB)

The immune function enhancing action of OHBFB was studied. Specifically, first, mononuclear cells were collected from the spleen of a Balb/c mouse (6-week old, female) and seeded at 2 mL per well in a 12-well plate at $2 \times 10^6$ cells/mL.

Subsequently, the mononuclear cells were cultured for 24 hours in a 10% FBS-RPMI medium including OHBFB at a final concentration of 0, 0.5, or 1 μM, and then RNA was recovered by TRI Reagent (Cosmo Bio Co.). Subsequently, after cDNA was synthesized, the expression levels of mRNA of a Toll-like receptor (TLR) 5 which is a receptor which recognizes bacterial flagellar proteins, TLR7 which is a receptor which recognizes a viral single-stranded RNA, and CCR5 which is involved in the proliferation of CD8-positive T cells were measured by a real-time PCR method. The expression levels of TLR5, TLR7, and CCR5 were normalized using the expression level of a β-actin gene which is a housekeeping gene.

For the PCR of TLR5, a sense primer whose nucleotide sequence is shown in SEQ ID NO: 1 and an antisense primer whose nucleotide sequence is shown in SEQ ID NO: 2 were used. For the PCR of TLR7, a sense primer whose nucleotide sequence is shown in SEQ ID NO: 3 and an antisense primer whose nucleotide sequence is shown in SEQ ID NO: 4 were used. For the PCR of CCR5, a sense primer whose nucleotide sequence is shown in SEQ ID NO: 5 and an antisense primer whose nucleotide sequence is shown in SEQ ID NO: 6 were used. In addition, for the PCR of the β-actin gene, a sense primer whose nucleotide sequence is shown in SEQ ID NO: 7 and an antisense primer whose nucleotide sequence is shown in SEQ ID NO: 8 were used.

FIG. 21 (a) to (c) are graphs showing the results of a real-time PCR. FIG. 21 (a) is a graph showing the measurement results of the expression level of TLR5. FIG. 21 (b) is a graph showing the measurement results of the expression level of TLR7. FIG. 21 (c) is a graph showing the measurement results of the expression level of CCR5. In FIG. 21 (a) to (c), the data are expressed in a mean value ± a standard deviation (n = 3 or 4). Furthermore, "*" indicates that a statistically significant difference exists at $p < 0.05$ for the results with 0 μM of OHBFB by a Dunnett's test. In addition, "**" indicates that a statistically significant difference exists at $p < 0.01$ for the results with 0 μM of OHBFB by a Dunnett's test.

As a result, it became clear that OHBFB promotes the expression of TLR5, TLR7, and CCR5 on spleen monocytes. That is, it became clear that OHBFB has an action of enhancing an ability to prevent bacterial infection and viral infection by promoting the expression of these molecules. Therefore, it can be said that OHBFB has an immune function enhancing action.

[Experimental Example 18]

(Arteriosclerosis Preventing Action/Thrombosis Preventing Action of OHBFB)

The arteriosclerosis preventing action/thrombosis preventing action of OHBFB were studied. Specifically, first, HUVEC which is a human umbilical vein endothelial cell was suspended in a 10% fetal bovine serum (FBS)-EGM-2 medium to be adjusted to $1.5 \times 10^5$ cells/mL, and the suspension was seeded at 2 mL per well in a 12-well plate. Subsequently, after pre-culturing for 24 hours, the medium was replaced with a 10% FBS-EGM-2 medium including OHBFB at final concentrations of 0, 1, and 5 μM, and the cells were further cultured for 24 hours.

Thereafter, the cells were recovered, RNA was purified, cDNA was synthesized, and the expression level of mRNA of a tissue factor pathway inhibitor (TFPI) which exhibits an arteriosclerosis-preventing action or a thrombus-preventing action was measured by a real-time PCR method.

The expression level of TFPI was normalized using the expression level of a β-actin gene which is a housekeeping gene. For the PCR of TFPI, a sense primer whose nucleotide sequence is shown in SEQ ID NO: 9 and an antisense primer whose nucleotide sequence is shown in SEQ ID NO: 10 were used. For the PCR of the β-actin gene, a sense primer whose nucleotide sequence is shown in SEQ ID NO: 11 and an antisense primer whose nucleotide sequence is shown in SEQ ID NO: 12 were used.

FIG. 22 is a graph showing the results of real-time PCR. In FIG. 22, the data are expressed in a mean value

± a standard deviation (n = 3). In addition, "**" indicates that a statistically significant difference exists at $p < 0.01$ for the results with 0 μM of OHBFB by a Dunnett's test.

As a result, it became clear that OHBFB promotes the expression of TFPI for human umbilical vein endothelial cells HUVEC. That is, it became clear that OHBFB has an arteriosclerosis preventing action and a thrombosis preventing action.

[Experimental Example 19]

(Muscular Atrophy Preventing Action of OHBFB)

The muscular atrophy preventing action of OHBFB was studied. Specifically, first, C2C12 which is a mouse myoblast cell line was suspended in a 10% fetal calf serum (FCS)-DMEM medium to be adjusted to $2 \times 10^4$ cells/mL, the suspension was seeded at 2 mL per well in a 12-well plate, and the cells were pre-cultured for 24 hours. Subsequently, the medium was replaced with a differentiation inducing medium with 0.5% FCS-DMEM and the cells were further cultured for 72 hours. After differentiation into myotube cells, the cells were replaced with a differentiation inducing medium containing OHBFB at a final concentration of 0, 1, or 2.5 μM, and further cultured for 48 hours.

Thereafter, RNA was extracted from the cells, cDNA was synthesized, and the expression levels of mRNA of Muscle RING-Finger Protein-1 (MuRF1), which is a type of muscular atrophy-related genes, were measured by real-time PCR. The expression level of MuRF1 was normalized using the expression level of a glyceraldehyde-3-phosphate dehydrogenase (GAPDH) gene, which is a housekeeping gene.

For the PCR of MuRF1, a sense primer whose nucleotide sequence is shown in SEQ ID NO: 13 and an antisense primer whose nucleotide sequence is shown in SEQ ID NO: 14 were used. In addition, for PCR of the GAPDH gene, a sense primer whose nucleotide sequence is shown in SEQ ID NO: 15 and an antisense primer whose nucleotide sequence is shown in SEQ ID NO: 16 were used.

FIG. 23 is a graph showing the results of a real-time PCR. In FIG. 23, the data are expressed in a mean value ± a standard deviation (n = 3). "n.s." indicates that there is no statistically significant difference for the results with 0 μM of OHBFB by a Dunnett's test, and "*" indicates that a statistically significant difference exists at $p < 0.05$ for the results with 0 μM of OHBFB by a Dunnett's test.

As a result, it became clear that OHBFB suppressed the expression of MuRF1 in skeletal muscle cells. That is, it became clear that OHBFB has a muscular atrophy suppressing action.

[Experimental Example 20]

(Anti-Inflammatory Action of OHBFB)

The anti-inflammatory action of OHBFB was studied. Specifically, first, RAW264.7 which is a mouse macrophage-like cell line was suspended in a 5% FBS-DMEM medium to be adjusted to $1 \times 10^4$ cells/mL, the suspension was seeded at 1 mL per well in a 24-well plate, and the cells were pre-cultured for 24 hours.

Subsequently, the medium was replaced with 5% FBS-DMEM containing OHBFB at a final concentration of 1 μM and the cells were further cultured for 1 hour. Furthermore, for comparison, a group to which OHBFB had not been added was also prepared. Subsequently, lipopolysaccharide (LPS) at a final concentration of 50 ng/mL was added thereto and the cells were further cultured for 24 hours. In addition, for comparison, a group to which LPS had not been added was also prepared.

Thereafter, the culture supernatant was recovered and the concentration of TNF-α which is an inflammatory cytokine was quantified by an ELISA method. In addition, the amount of protein in the culture supernatant was measured and the amount of TNF-α per unit protein amount was calculated.

FIG. 24 is a graph showing the calculation results of the amount of a TNF-α. In FIG. 24, the data are expressed in a mean value ± a standard deviation (n = 3). In addition, "***" indicates that a statistically significant difference exists at $p < 0.001$ by a Tukey's test.

As a result, it became clear that OHBFB suppressed LPS-induced TNF-α expression induction in macrophages. That is, it became clear that OHBFB has anti-inflammatory properties.

[Experimental Example 21]

(Anti-Allergic Action of OHBFB)

The anti-allergic action of OHBFB was studied. Specifically, the effect of OHBFB on the release of a β-hexosaminidase from basophil cells was studied.

First, 2 × $10^5$ KU812 which are human basophil cells were suspended in 100 mL of a Tyrode's solution. Subsequently, 100 mL of the cell suspension was mixed with 100 mL of a Tyrode's solution including $CaCl_2$ at a final concentration of 1 mM, calcium ionophore A23187 at a final concentration of 50 mM, and OHBFB at a final concentration of 0, 5, or 25 μM, and kept at a constant temperature of 37°C for 20 minutes. Thereafter, the mixture was left to stand on ice for 5 minutes to stop the reaction.

Subsequently, the mixture was centrifuged at 4°C and 200 × g for 5 minutes, and the supernatant was recovered. 50 mL of a substrate solution (0.5 mM p-nitrophenyl-N-acetyl-b-D-glucosaminide) was added to 50 mL of the recovered supernatant and reacted at 37°C for 60 minutes.

Subsequently, 200 mL of a reaction stop solution (0.1 M sodium bicarbonate buffer, pH 10) was added thereto, an absorbance at a wavelength of 405 nm was measured, and a β-hexosaminidase activity in the supernatant was measured.

Furthermore, Triton-X at a final concentration of 1% was added to the cell suspension and the same operation as described above was performed to measure an intracellular β-hexosaminidase activity. Subsequently, the release rate of the β-hexosaminidase was calculated by Formula (F1).

β-Hexosaminidase release rate (%) = β-Hexosaminidase activity in supernatant (absorbance at wavelength of 405 nm)/β-Hexosaminidase activity in cells (absorbance at wavelength of 405 nm) × 100 ... (F1)

FIG. 25 is a graph showing the calculation results of the release rate of the β-hexosaminidase. In FIG. 25, the data are expressed in a mean value ± a standard deviation (n = 3). In addition, "**" indicates that a statistically significant difference exists at $p < 0.01$ for the results with 0 μM of OHBFB by a Dunnett's test.

As a result, it became clear that OHBFB inhibited the degranulation in basophil cells. That is, it became clear that OHBFB has an anti-allergic action.

Industrial Applicability

According to the present invention, a 67LR agonist which is more potent than EGCG can be provided.

## Claims

1. A 67 kDa laminin receptor (67LR) agonist comprising an oolong tea polymerized polyphenol or a derivative thereof as an active ingredient.

2. The 67LR agonist according to claim 1,
wherein the oolong tea polymerized polyphenol or a derivative thereof is a compound represented by Formula (1) or (2),

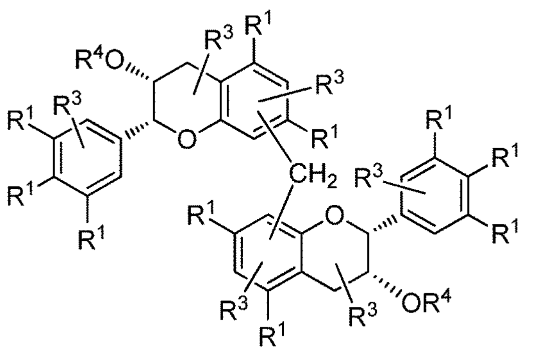

(1)

[in Formula (1), each of $R^1$ independently represents a hydrogen atom, a hydroxyl group, an alkyl group having 1 to 3 carbon atoms or $-OR^2$ (in which $R^2$ represents a group formed by removing one hydrogen atom from glucose, mannose, xylose, galactose, or maltose), each of $R^3$ is independently absent or represents a halogen atom or an alkyl group having 1 to 3 carbon atoms, and each of $R^4$ independently represents a hydrogen atom or a group

represented by Formula (1A)]

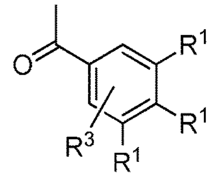

(1A)

[in Formula (1A), $R^1$ and $R^3$ have the same definitions as in Formula (1)]

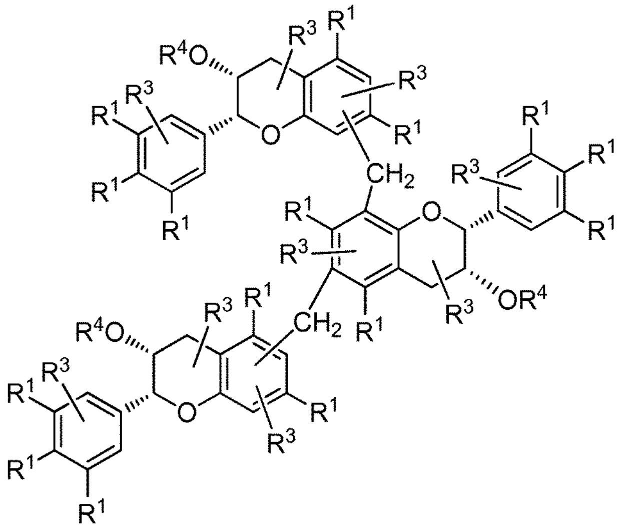

(2)

[in Formula (2), $R^1$, $R^3$, and $R^4$ have the same definitions as in Formula (1)].

**3.** The 67LR agonist according to claim 1 or 2,
wherein the oolong tea polymerized polyphenol or a derivative thereof is a compound represented by any of Formulae (3) to (6),

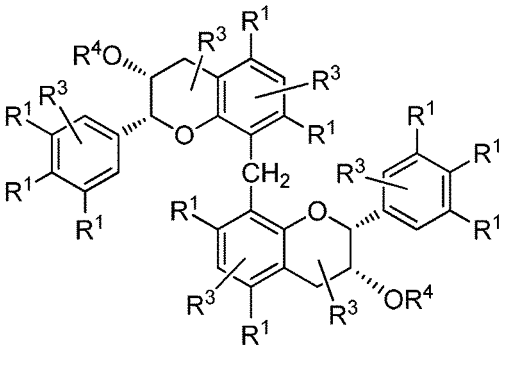

(3)

[in Formula (3), $R^1$, $R^3$, and $R^4$ have the same definitions as in Formula (1)]

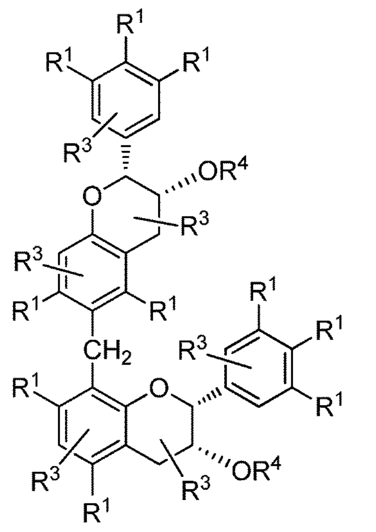

(4)

[in Formula (4), $R^1$, $R^3$, and $R^4$ have the same definitions as in Formula (1)]

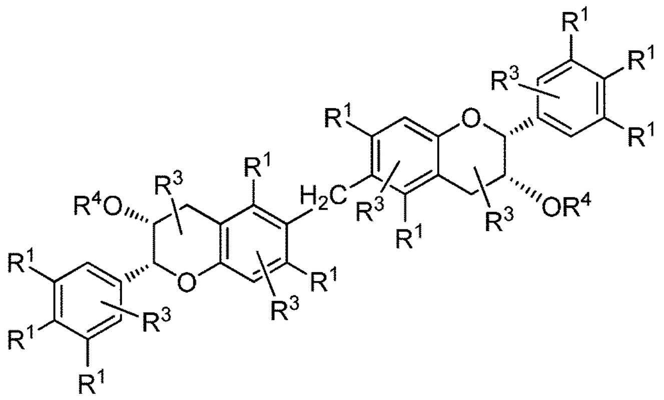

(5)

[in Formula (5), $R^1$, $R^3$, and $R^4$ have the same definitions as in Formula (1)]

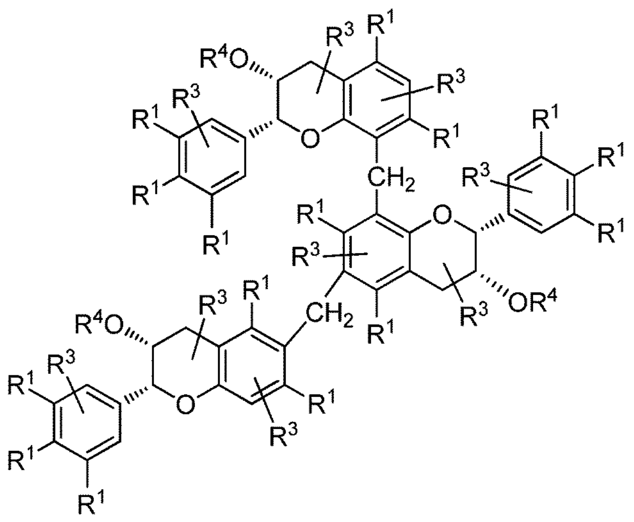

(6)

[in Formula (6), $R^1$, $R^3$, and $R^4$ have the same definitions as in Formula (1)].

4. The 67LR agonist according to any one of claims 1 to 3, which is used for preventing or treating a 67LR-related disease.

5. The 67LR agonist according to claim 4,
wherein the 67LR-related disease is cancer, allergy, obesity, arteriosclerosis, inflammation, muscular atrophy, hypercholesterolemia, thrombosis, or immunodeficiency.

6. A 67LR agonist composition comprising:

the 67LR agonist according to any one of claims 1 to 3; and
a phosphodiesterase inhibitor, or a flavanone or a glycoside thereof.

7. The 67LR agonist composition according to claim 6,
which is used for preventing or treating a 67LR-related disease.

8. The 67LR agonist composition according to claim 7,
wherein the 67LR-related disease is cancer, allergy, obesity, arteriosclerosis, inflammation, muscular atrophy, hypercholesterolemia, thrombosis, or immunodeficiency.

9. A food composition for preventing or ameliorating a 67LR-related disease, comprising the 67LR agonist according to any one of claims 1 to 3.

10. The food composition for preventing or ameliorating a 67LR-related disease according to claim 9, further comprising a phosphodiesterase inhibitor, or a flavanone or a glycoside thereof.

11. A method for preventing or ameliorating a 67LR-related disease, comprising a step of ingesting the food composition according to claim 9 or 10 into a human or animal (excluding medical practice on humans).

12. The method for preventing or ameliorating according to claim 11, wherein the 67LR-related disease is cancer, allergy, obesity, arteriosclerosis, inflammation, muscular atrophy, hypercholesterolemia, thrombosis, or immunodeficiency.

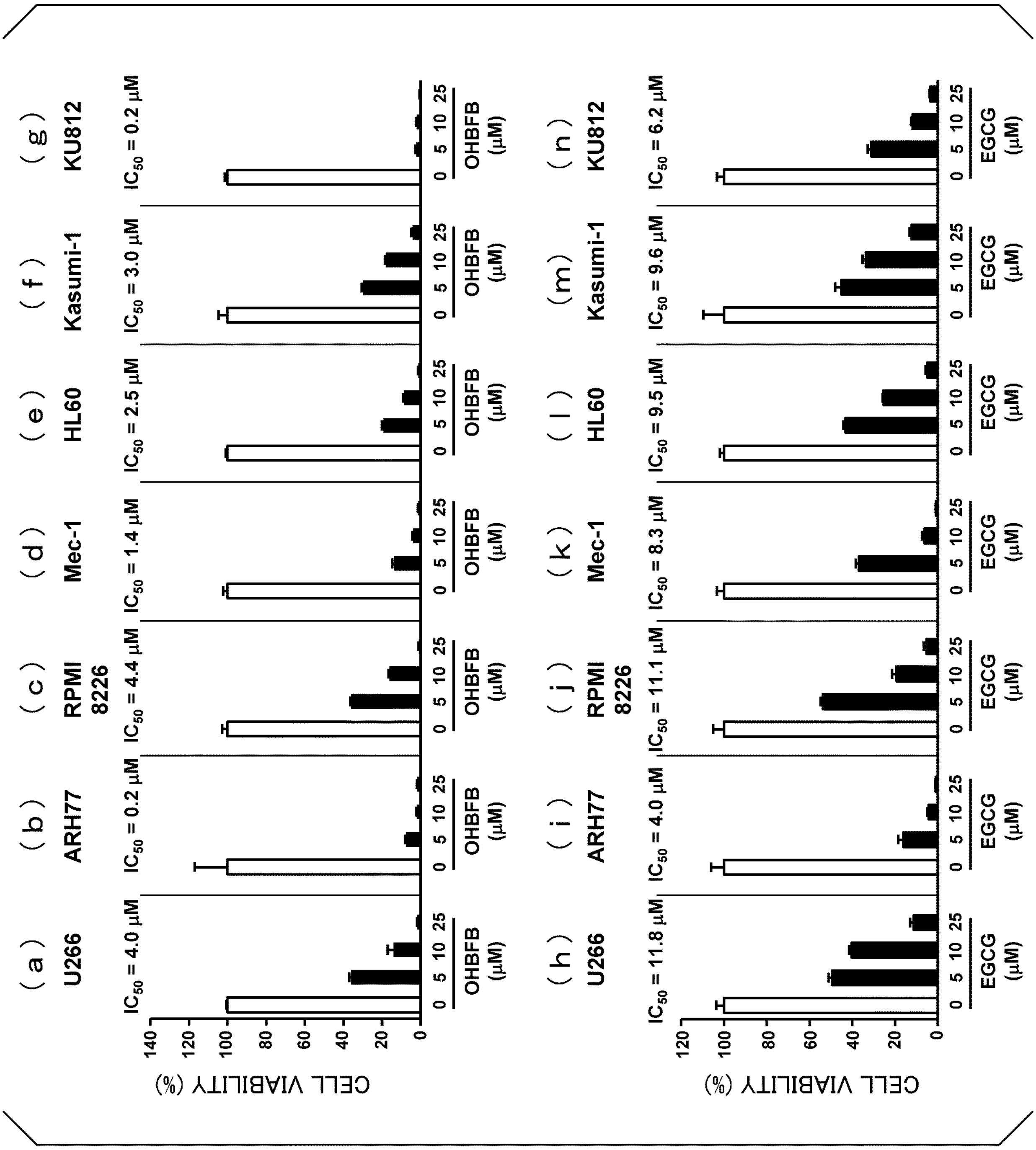

(a) U266 $IC_{50}$ = 4.0 μM
(b) ARH77 $IC_{50}$ = 0.2 μM
(c) RPMI 8226 $IC_{50}$ = 4.4 μM
(d) Mec-1 $IC_{50}$ = 1.4 μM
(e) HL60 $IC_{50}$ = 2.5 μM
(f) Kasumi-1 $IC_{50}$ = 3.0 μM
(g) KU812 $IC_{50}$ = 0.2 μM
(h) U266 $IC_{50}$ = 11.8 μM
(i) ARH77 $IC_{50}$ = 4.0 μM
(j) RPMI 8226 $IC_{50}$ = 11.1 μM
(k) Mec-1 $IC_{50}$ = 8.3 μM
(l) HL60 $IC_{50}$ = 9.5 μM
(m) Kasumi-1 $IC_{50}$ = 9.6 μM
(n) KU812 $IC_{50}$ = 6.2 μM
CELL VIABILITY (%)
0 5 10 25
OHBFB (μM)
EGCG (μM)


FIG. 1

FIG. 2

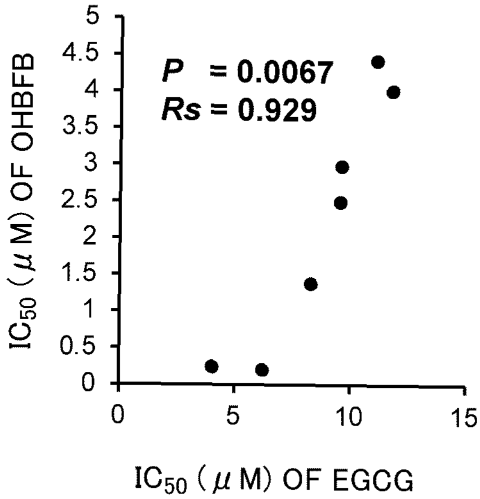

P = 0.0067
Rs = 0.929
IC50 (μM) OF OHBFB
IC50 (μM) OF EGCG
0
0.5
1
1.5
2
2.5
3
3.5
4
4.5
5
0
5
10
15


FIG. 3

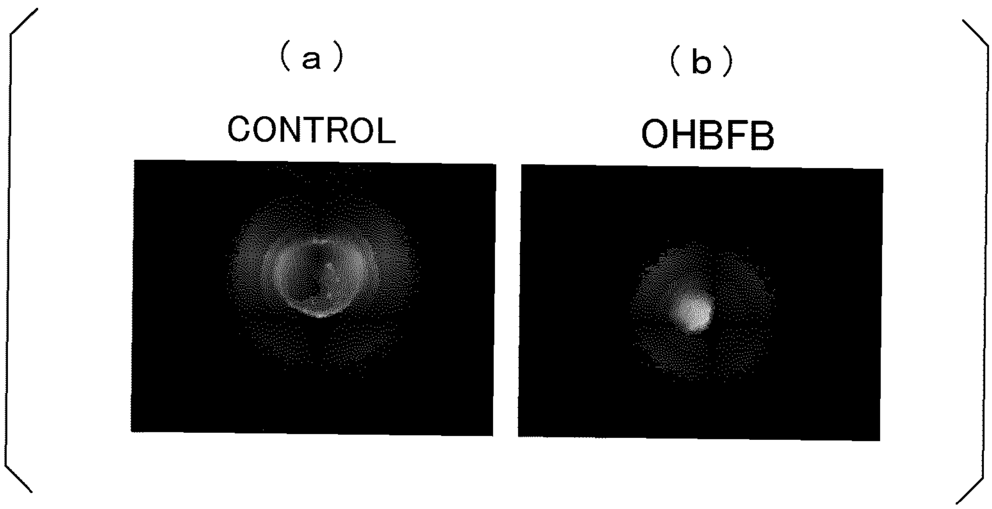

(a)
CONTROL
(b)
OHBFB

FIG. 4

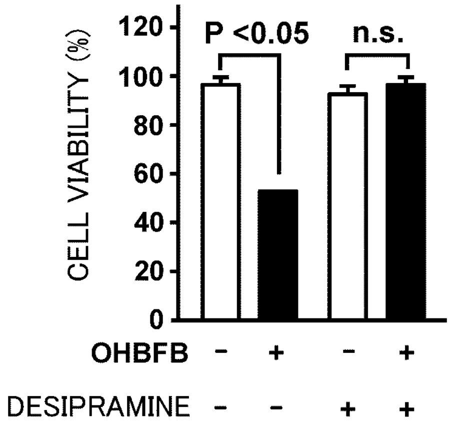


FIG. 5

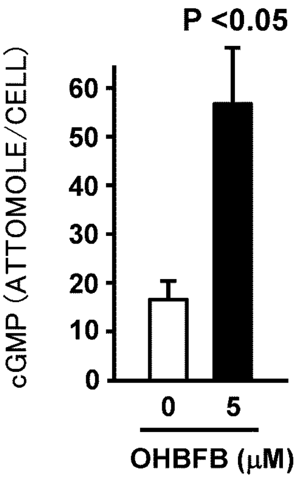


FIG. 6

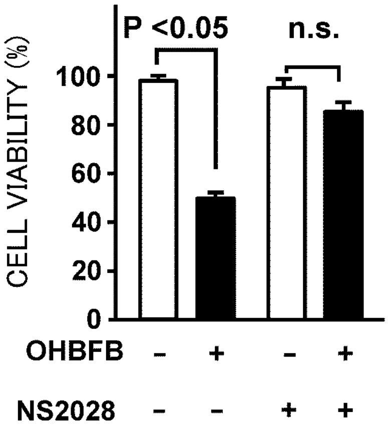

FIG. 7

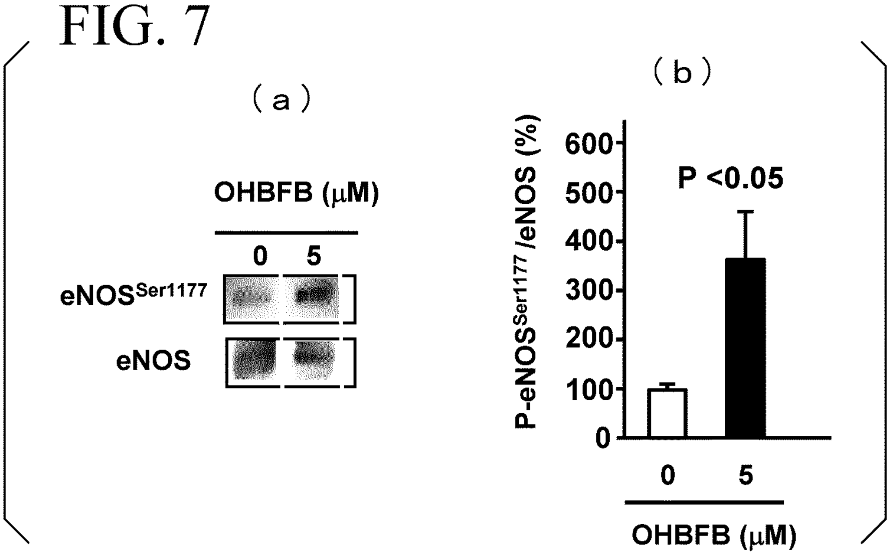


FIG. 8

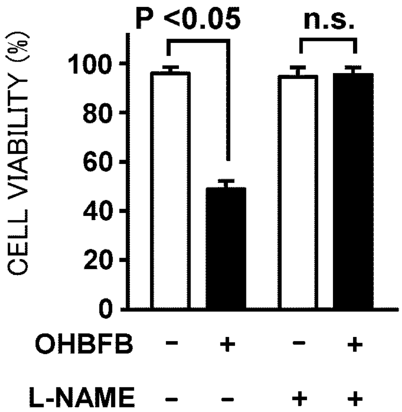


FIG. 9

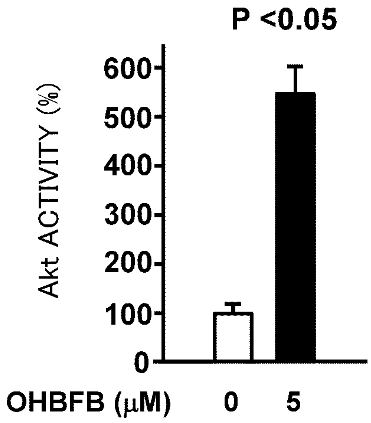

FIG. 10

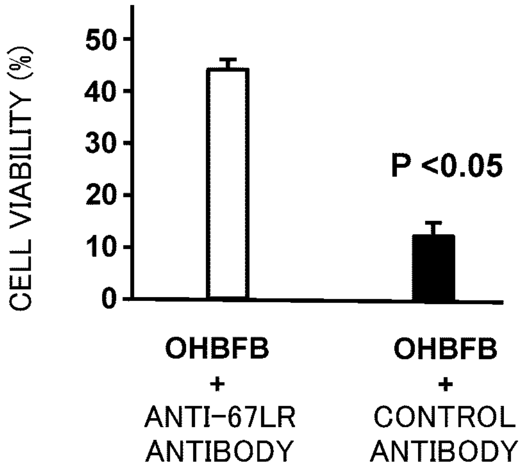
CELL VIABILITY (%)
50
40
30
20
10
0
P <0.05
OHBFB
+
ANTI-67LR
ANTIBODY
OHBFB
+
CONTROL
ANTIBODY

FIG. 11

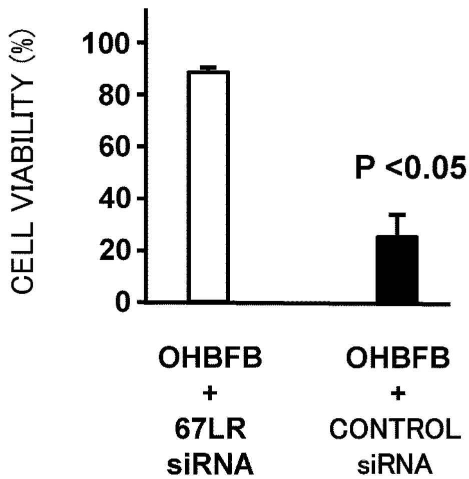
CELL VIABILITY (%)
100
80
60
40
20
0
P <0.05
OHBFB
+
67LR
siRNA
OHBFB
+
CONTROL
siRNA

FIG. 12

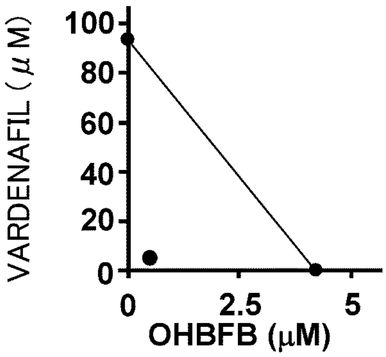
VARDENAFIL（μM）
100
80
60
40
20
0
0
2.5
5
OHBFB (μM)

FIG. 13

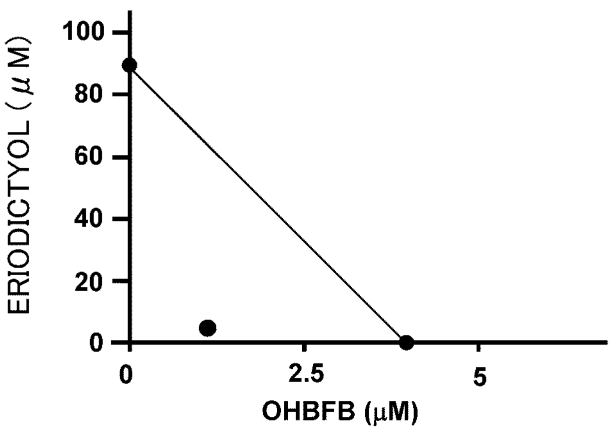
ERIODICTYOL（μM）
100
80
60
40
20
0
0
2.5
5
OHBFB (μM)

FIG. 14

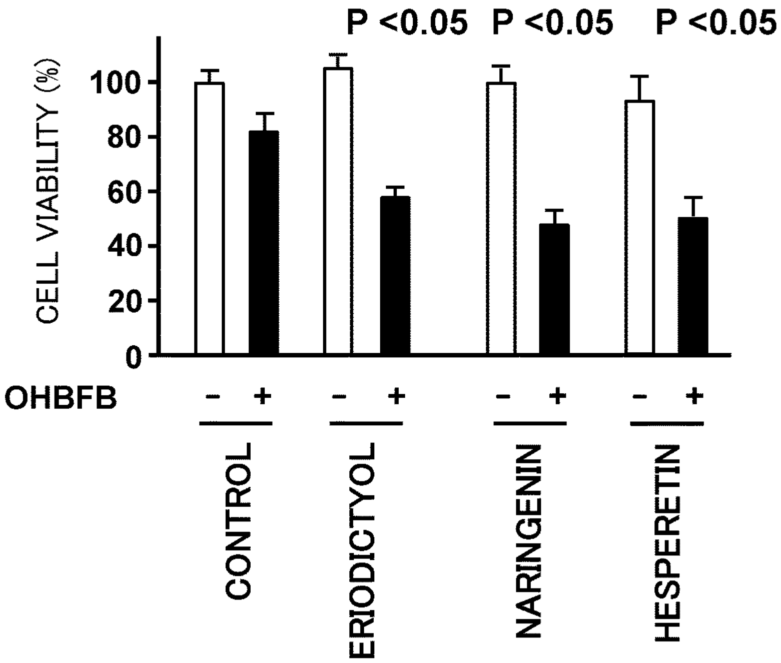
P <0.05
P <0.05
P <0.05
CELL VIABILITY (%)
100
80
60
40
20
0
OHBFB
- +
- +
- +
- +
CONTROL
ERIODICTYOL
NARINGENIN
HESPERETIN

FIG. 15

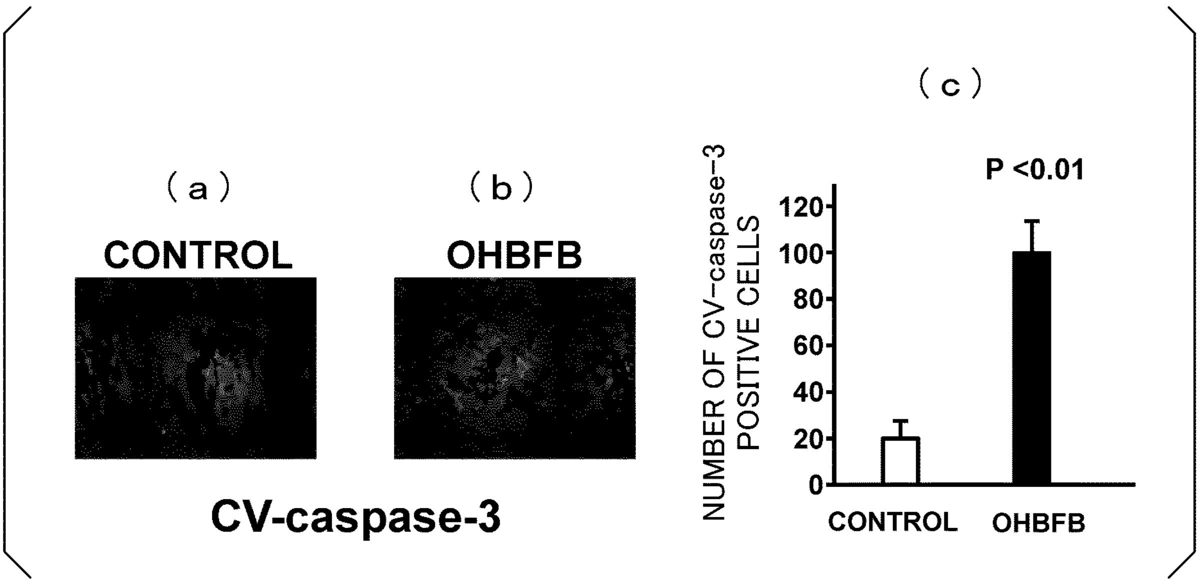
(a)
CONTROL
(b)
OHBFB
CV-caspase-3
(c)
P <0.01
NUMBER OF CV-caspase-3 POSITIVE CELLS
120
100
80
60
40
20
0
CONTROL
OHBFB

FIG. 16

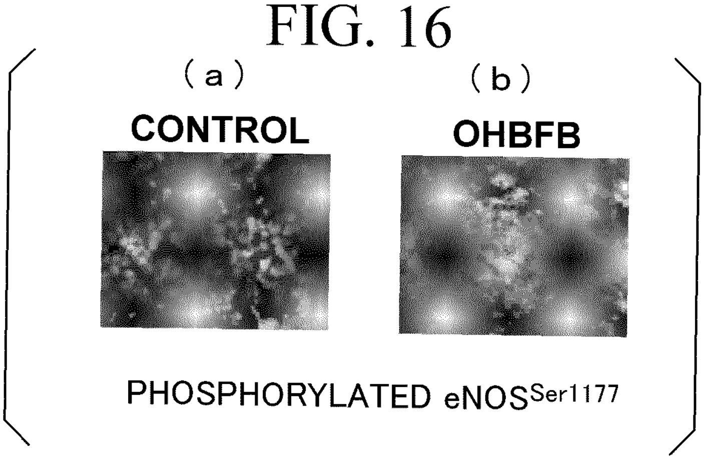


FIG. 17

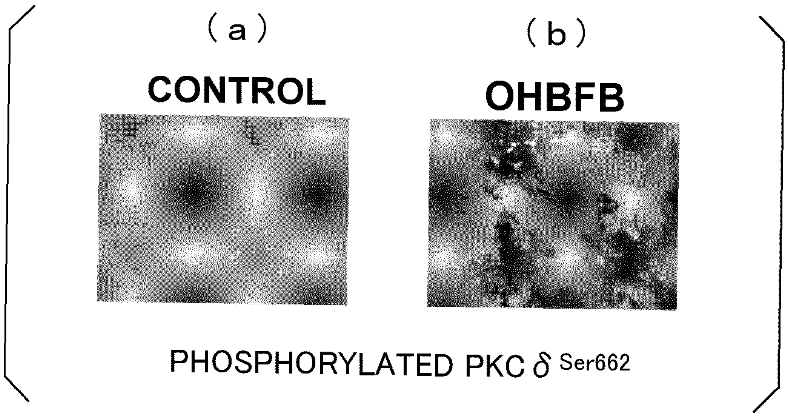


FIG. 18

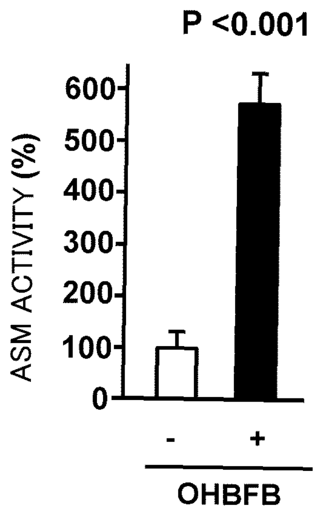

FIG. 19

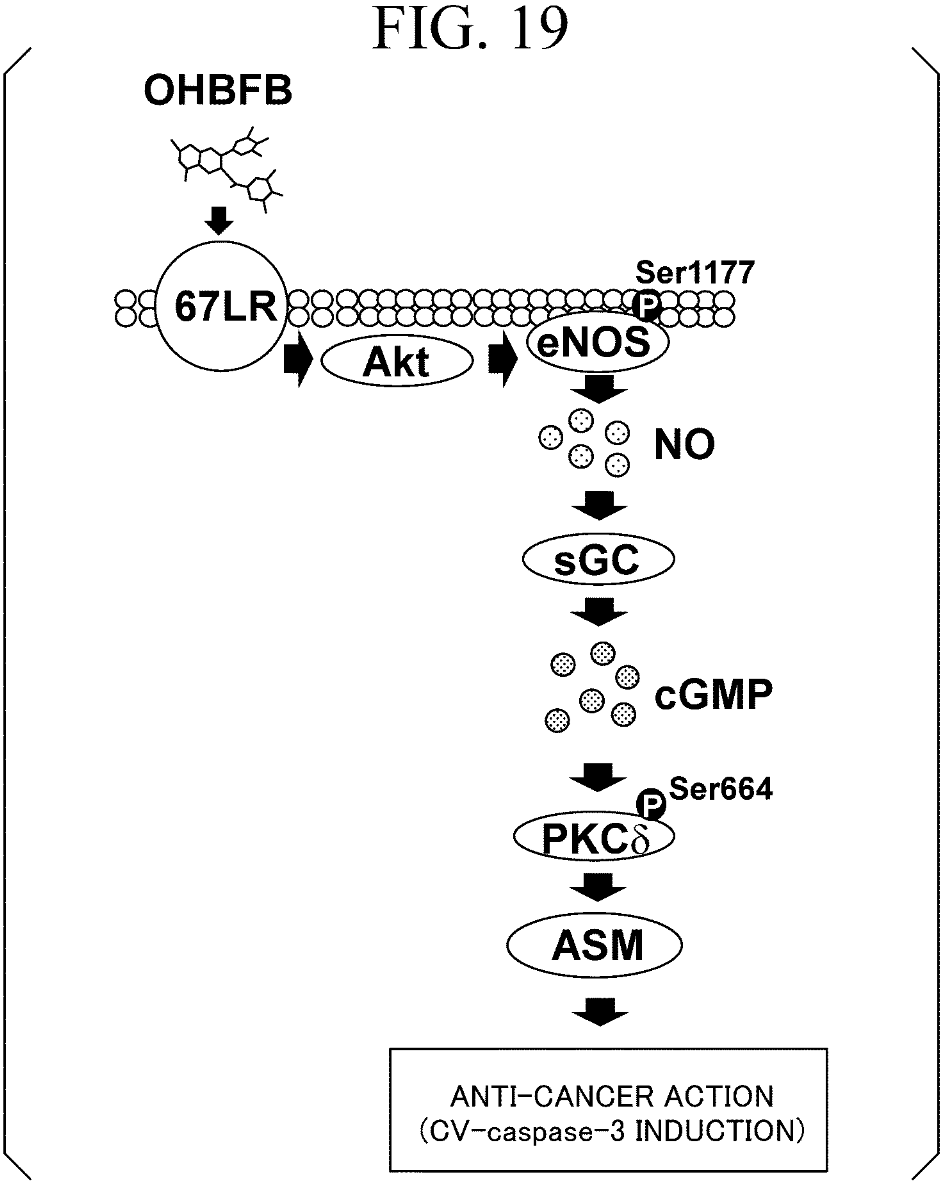
OHBFB
67LR
Akt
Ser1177
P
eNOS
NO
sGC
cGMP
P
Ser664
PKCδ
ASM
ANTI-CANCER ACTION
(CV-caspase-3 INDUCTION)

FIG. 20

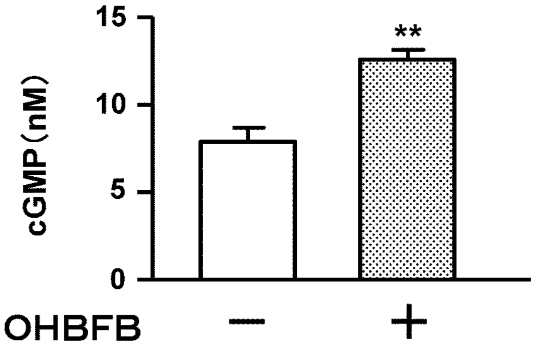
15
10
5
0
cGMP (nM)
**
OHBFB
−
+

FIG. 21

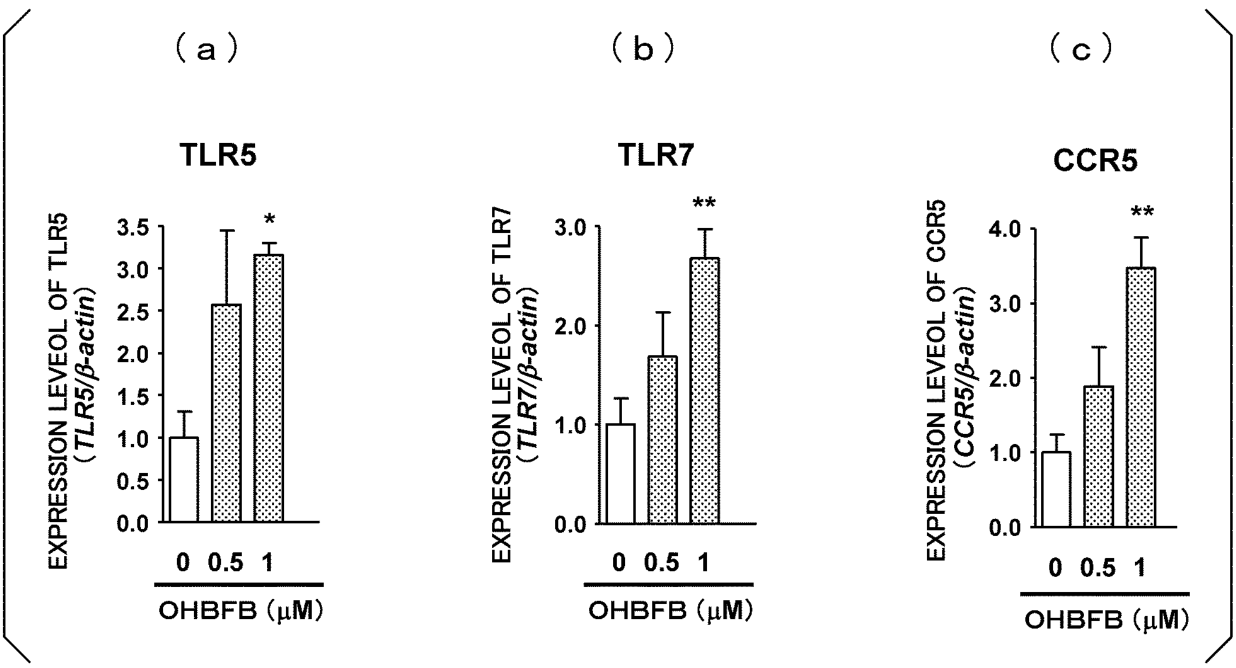
(a)
TLR5
EXPRESSION LEVEOL OF TLR5
(TLR5/β-actin)
3.5
3.0
2.5
2.0
1.5
1.0
0.5
0.0
*
0
0.5
1
OHBFB (μM)
(b)
TLR7
EXPRESSION LEVEOL OF TLR7
(TLR7/β-actin)
3.0
2.0
1.0
0.0
**
0
0.5
1
OHBFB (μM)
(c)
CCR5
EXPRESSION LEVEOL OF CCR5
(CCR5/β-actin)
4.0
3.0
2.0
1.0
0.0
**
0
0.5
1
OHBFB (μM)

FIG. 22

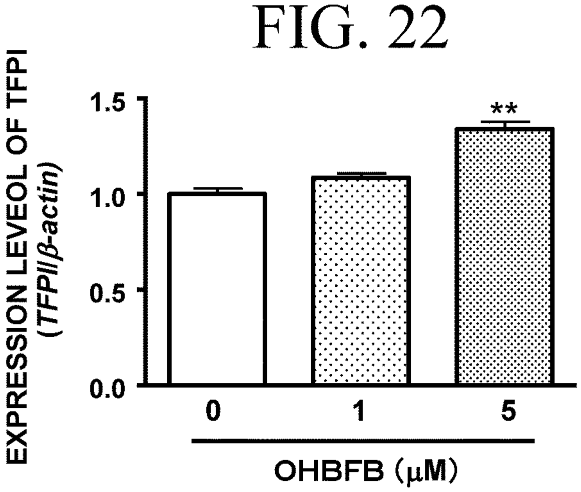
EXPRESSION LEVEOL OF TFPI
(TFPI/β-actin)
1.5
1.0
0.5
0.0
**
0
1
5
OHBFB (μM)

FIG. 23

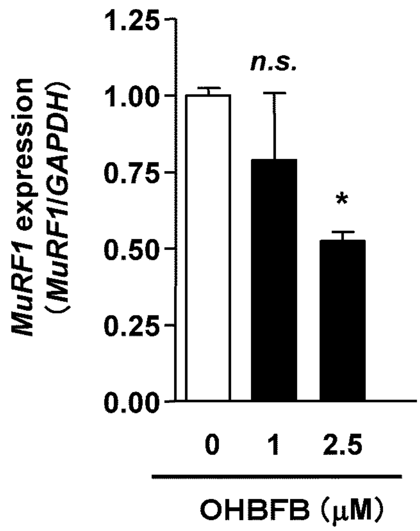
MuRF1 expression
(MuRF1/GAPDH)
1.25
1.00
0.75
0.50
0.25
0.00
n.s.
*
0
1
2.5
OHBFB (μM)

FIG. 24

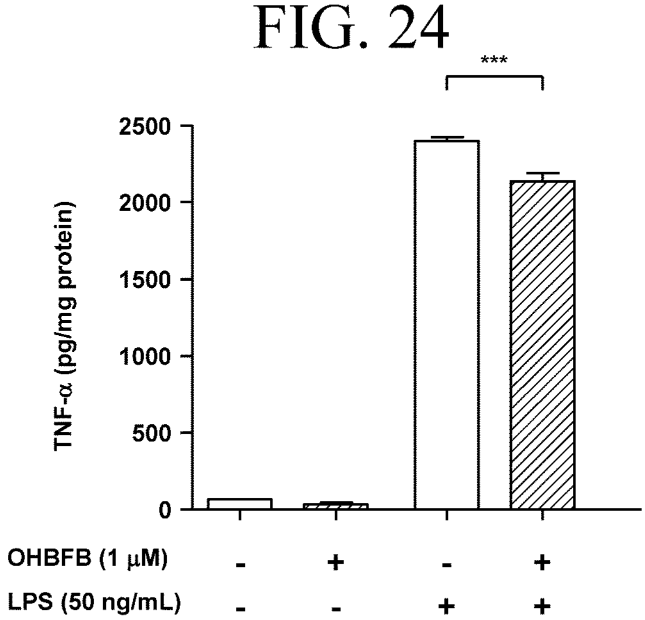
***
TNF-α (pg/mg protein)
2500
2000
1500
1000
500
0
OHBFB (1 μM) - + - +
LPS (50 ng/mL) - - + +

FIG. 25

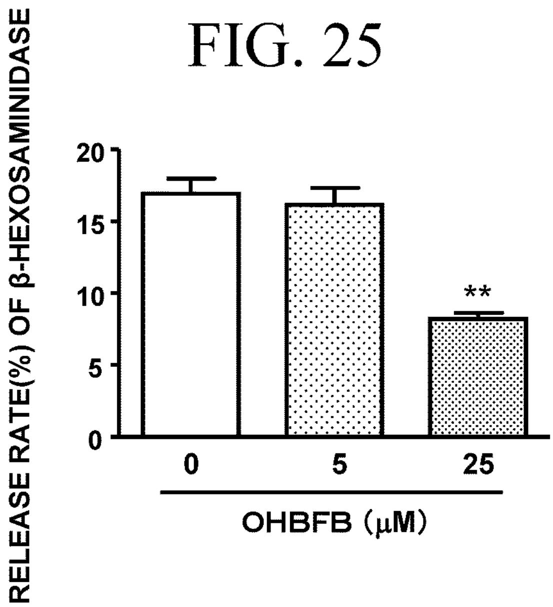
RELEASE RATE(%) OF β-HEXOSAMINIDASE
20
15
10
5
0
**
0
5
25
OHBFB (μM)

**INTERNATIONAL SEARCH REPORT**

International application No.
PCT/JP2017/041248

A. CLASSIFICATION OF SUBJECT MATTER
Int.Cl. [see extra sheet]

According to International Patent Classification (IPC) or to both national classification and IPC

B. FIELDS SEARCHED

Minimum documentation searched (classification system followed by classification symbols)
Int.Cl. A61K31/353, A61P3/04, A61P3/06, A61P7/02, A61P9/10, A61P21/00, A61P29/00, A61P35/00, A61P35/02, A61P37/04, A61P37/08, A61P43/00, A23L33/105, C12N15/09

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y | 吉本孝憲，ウーロンホモビスフラバンＢは67LRを介して多発性骨髄腫細胞にアポトーシスを誘導する，JSoFF Letter [online], no. 71, June, 15 June 2017, Internet, p. 11, <URL:http://nodaiweb.university.jp/jsoff/jsoffletter71.pdf>, p. 11, entire text, non-official translation (YOSHIMOTO, Takanori, Oolonghomobisflavan B induces apoptosis in multiple myeloma cells via 67LR) | 1-5, 9, 11-12<br>1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31.01.2018 | 13.02.2018 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2017/041248

C (Continuation). DOCUMENTS CONSIDERED TO BE RELEVANT

| Category* | Citation of document, with indication, where appropriate, of the relevant passages | Relevant to claim No. |
|---|---|---|
| Y | KUMAZOE, M., The Journal of Clinical Investigation, 67-kDa laminin receptor increases cGMP to induce cancer-selective apoptosis, February 2013, vol. 123, no. 2, pp. 787-799, fig. 6(F) | 1-12 |
| Y | WO 2011/162320 A1 (KYUSHU UNIVERSITY) 29 December 2011, claims, paragraphs [0056]-[0061], [0071]-[0073], [0103], [0104] & US 2013/0172356 A1, claims, paragraphs [0111]-[0118], [0131]-[0134], [0177]-[0179] & EP 2586441 A1 | 6-8, 10-12 |
| Y | WO 2015/199169 A1 (KYUSHU UNIVERSITY) 30 December 2015, claims, examples 1, 2, 4, 9, 10 & US 2017/0156361 A1, claims, examples 1, 2, 4, 9, 10 | 6-8, 10-12 |
| X | JP 2005-75790 A (HASHIMOTO, Fumio et al.) 24 March 2005, claims, paragraph [0036], examples 8-13, 17 (Family: none) | 1-5, 9, 11-12 |
| X | JP 2007-119412 A (KAGOSHIMA UNIV) 17 May 2007, claims, paragraph [0068] (Family: none) | 1-5, 9, 11-12 |
| X | JP 2005-336117 A (SUNTORY LTD.) 08 December 2005, claims, paragraphs [0013], [0029], example 4 & US 2008/0275258 A1, claims, paragraphs [0037], [0049], examples 1, 2 & WO 2005/116005 A1 & EP 1754702 A1 & CN 1989123 A & KR 10-2011-0016507 A | 1-5, 9, 11-12 |
| X | 中村淳一 他, 特定保健用食品「黒烏龍茶 OTPP」の継続摂取による内蔵脂肪低減効果とその安全性, 薬理と治療, June 2007, vol. 35, no. 6, pp. 661-671, p. 662, right column, [2. 試験飲料], p. 671, left column, ll. 10-19, (NAKAMURA, Junichi et al., Lowering effects on visceral fat of the OTPP (Oolong Tea Polymerized Polyphenols) enriched oolong tea (FOSHU "KURO-Oolong Tea OTPP") in over weight volunteers, Japanese Pharmacology & Therapeutics), non-official translation (2. Test beverage) | 1-5, 9, 11-12 |
| X | JP 2001-39881 A (ASAHI BREWERIES LTD.) 13 February 2001, claims, paragraphs [0014], [0016], [0018], [0028] (Family: none) | 1-5, 9, 11-12 |
| X | JP 8-3054 A (LOHMANN KOGYO KK) 09 January 1996, claims, paragraph [0002], example 2 (Family: none) | 1-5, 9, 11-12 |
| X | JP 10-77231 A (SUNTORY LTD.) 24 March 1998, claims, paragraphs [0013], [0015], examples 1, 2 (Family: none) | 1-5, 9, 11-12 |

Form PCT/ISA/210 (continuation of second sheet) (January 2015)

**INTERNATIONAL SEARCH REPORT**

International application No.
PCT/JP2017/041248

C (Continuation). DOCUMENTS CONSIDERED TO BE RELEVANT

| Category* | Citation of document, with indication, where appropriate, of the relevant passages | Relevant to claim No. |
|---|---|---|
| X | 松井陽吉, 茶系飲料について ウーロン茶の魅力, 日本食生活学会誌, 2000, vol. 11, no. 1, pp. 2-15, p. 10, right column, table 17, p. 11, left column (2), (MATSUI, Yokichi, Journal for the Integrated Study of Dietary Habits), non-official translation (On tea-base beverage • The charm of oolong tea) | 1-5, 9, 11-12 |

Form PCT/ISA/210 (continuation of second sheet) (January 2015)

**INTERNATIONAL SEARCH REPORT**

International application No.
PCT/JP2017/041248

(Continuation of Box No. A)

CLASSIFICATION OF SUBJECT MATTER
A61K31/353(2006.01)i, A61P3/04(2006.01)i, A61P3/06(2006.01)i, A61P7/02(2006.01)i, A61P9/10(2006.01)i, A61P21/00(2006.01)i, A61P29/00(2006.01)i, A61P35/00(2006.01)i, A61P35/02(2006.01)i, A61P37/04(2006.01)i, A61P37/08(2006.01)i, A61P43/00(2006.01)i, A23L33/105(2016.01)n, C12N15/09(2006.01)n

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017151708 A
- WO 2011162320 A
- WO 2015199169 A


### Non-patent literature cited in the description


- **KHAN N et al.** Targeting multiple signaling pathways by green tea polyphenol (-)-epigallocatechin-3-gallate. *Cancer res.,* 2006, vol. 66 (5), 2500-2505
- **SHANAFELT TD et al.** Phase I trial of daily oral Polyphenon E in patients with asymptomatic Rai stage 0 to II chronic lymphocytic leukemia. *J. Clin.Oncol.,* 2009, vol. 27 (23), 3808-3814
- **TACHIBANA H et al.** A receptor for green tea polyphenol EGCG. *Nat.Struct. Mol. Biol.,* 2004, vol. 11 (4), 380-381

SEQUENCE LISTING

<110> KYUSHU UNIVERSITY

<120> 67 kDa LAMININ RECEPTOR AGONIST AND USES THEREOF

<130> PC-24705

<150> JP2017-151708
<151> 2017-08-04

<160> 16

<170> PatentIn version 3.5

<210> 1
<211> 26
<212> DNA
<213> Mus musculus

<400> 1
aaggacaagg tctggagtag tttgga 26

<210> 2
<211> 22
<212> DNA
<213> Mus musculus

<400> 2
gcctgtttcg ggaactgtag tg 22

<210> 3
<211> 22
<212> DNA
<213> Mus musculus

<400> 3
cttgcaactg tgatgctgtg tg 22

<210> 4
<211> 23
<212> DNA
<213> Mus musculus

<400> 4
acctttgtgt gctcctggac cta 23

<210> 5
<211> 25
<212> DNA
<213> Mus musculus

<400> 5
gttaggttga aatacccatt gagga 25

<210> 6
<211> 22
<212> DNA

<213> Mus musculus

<400> 6
ggatttaggt ctgaaggcag ga 22

<210> 7
<211> 24
<212> DNA
<213> Mus musculus

<400> 7
catccgtaaa gacctctatg ccaa 24

<210> 8
<211> 19
<212> DNA
<213> Mus musculus

<400> 8
atggagccac cgatccaca 19

<210> 9
<211> 25
<212> DNA
<213> Homo sapiens

<400> 9
actcagctaa ttcacactga tttcc 25

<210> 10
<211> 23
<212> DNA
<213> Homo sapiens

<400> 10
catcagctgt accctgactt cac 23

<210> 11
<211> 19
<212> DNA
<213> Homo sapiens

<400> 11
tggcacccag cacaatgaa 19

<210> 12
<211> 25
<212> DNA
<213> Homo sapiens

<400> 12
ctaagtcata gtccgcctag aagca 25

<210> 13
<211> 20
<212> DNA

```
<213>  Mus musculus

<400>  13
tgaggtgcct acttgctcct                                                  20


<210>  14
<211>  20
<212>  DNA
<213>  Mus musculus

<400>  14
tcacctggtg gctattctcc                                                  20


<210>  15
<211>  20
<212>  DNA
<213>  Mus musculus

<400>  15
tgtgtccgtc gtggatctga                                                  20


<210>  16
<211>  21
<212>  DNA
<213>  Mus musculus

<400>  16
ttgctgttga agtcgcagga g                                                21
```